# EUROPEAN PATENT SPECIFICATION

(11) **EP 3 917 912 B1**
(45) Date of publication and mention of the grant of the patent: **21.05.2025**
(21) Application number: 20710338.3
(22) Date of filing: 28.01.2020
(51) Int. Cl.: C07D 213/82, C07D 237/24, C07D 401/12, C07D 403/12, C07D 405/14, C07D 417/14, C07D 413/12, C07B 59/00, A61K 31/44, A61K 31/50, A61K 31/501

(54) **AMIDE-DISUBSTITUTED PYRIDINE OR PYRIDAZINE COMPOUNDS**
AMID-DISUBSTITUIERTE PYRIDIN- ODER PYRIDAZIN-VERBINDUNGEN
COMPOSÉS DE PYRIDINE OU DE PYRIDAZINE À DISUBSTITUTION AMIDE

(30) Priority: 30.01.2019 US 201962798566 P
(43) Date of publication of application: 08.12.2021
(73) Proprietor: Bristol-Myers Squibb Company, Princeton, NJ 08543 (US)
(72) Inventor: SHERWOOD, Trevor C., Princeton, New Jersey 08543 (US); YANG, Michael G., Princeton, New Jersey 08543 (US); GILMORE, John L., Princeton, New Jersey 08543 (US); ZHANG, Yanlei, Princeton, New Jersey 08540 (US); XIAO, Zili, Princeton, New Jersey 08543 (US); LIU, Qingjie, Princeton, New Jersey 08543 (US)
(74) Representative: Mewburn Ellis LLP
(86) International application number: PCT/US2020/015291
(87) International publication number: WO 2020/159904

(56) References cited:
- WO-A1-2014/074661
- WO-A1-2015/069310
- WO-A1-2018/071794
- WO-A1-2020/086616
- WO-A1-2020/156311

## Description

### FIELD OF THE INVENTION

This invention relates to compounds useful in the modulation of IL-12, IL-23 and/or IFNα by acting on Tyk-2 to cause signal transduction inhibition. Provided herein are amide-substituted heterocyclic compounds, compositions comprising such compounds, and such compounds for use. The invention further pertains to pharmaceutical compositions containing at least one compound according to the invention that are useful for the treatment of conditions related to the modulation of IL-12, IL-23 and/or IFNα in a mammal.

### BACKGROUND OF THE INVENTION

The heterodimeric cytokines interleukin (IL)-12 and IL-23, which share a common p40 subunit, are produced by activated antigen-presenting cells and are critical in the differentiation and proliferation of Th1 and Th17 cells, two effector T cell lineages which play key roles in autoimmunity. IL-23 is composed of the p40 subunit along with a unique p19 subunit. IL-23, acting through a heterodimeric receptor composed of IL-23R and IL-12Rβ1, is essential for the survival and expansion of Th17 cells which produce pro-inflammatory cytokines such as IL-17A, IL-17F, IL-6 and TNF-α (McGeachy, M.J. et al., "The link between IL-23 and Th17 cell-mediated immune pathologies", Semin. Immunol., 19:372-376 (2007)). These cytokines are critical in mediating the pathobiology of a number of autoimmune diseases, including rheumatoid arthritis, multiple sclerosis, inflammatory bowel disease, and lupus. IL-12, in addition to the p40 subunit in common with IL-23, contains a p35 subunit and acts through a heterodimeric receptor composed of IL-12Rβ1 and IL-12Rβ2. IL-12 is essential for Th1 cell development and secretion of IFNγ, a cytokine which plays a critical role in immunity by stimulating MHC expression, class switching of B cells to IgG subclasses, and the activation of macrophages (Gracie, J.A. et al., "Interleukin-12 induces interferon-gamma-dependent switching of IgG alloantibody subclass", Eur. J. Immunol., 26:1217-1221 (1996); Schroder, K. et al., "Interferon-gamma: an overview of signals, mechanisms and functions", J. Leukoc. Biol., 75(2):163-189 (2004)).

The importance of the p40-containing cytokines in autoimmunity is demonstrated by the discovery that mice deficient in either p40, p19, or IL-23R are protected from disease in models of multiple sclerosis, rheumatoid arthritis, inflammatory bowel disease, lupus and psoriasis, among others (Kyttaris, V.C. et al., "Cutting edge: IL-23 receptor deficiency prevents the development of lupus nephritis in C57BL/6-lpr/lpr mice", J. Immunol., 184:4605-4609 (2010); Hong, K. et al., "IL-12, independently of IFN-gamma, plays a crucial role in the pathogenesis of a murine psoriasis like skin disorder", J. Immunol., 162:7480-7491 (1999); Hue, S. et al., "Interleukin-23 drives innate and T cell-mediated intestinal inflammation", J. Exp. Med., 203:2473-2483 (2006); Cua, D.J. et al., "Interleukin-23 rather than interleukin-12 is the critical cytokine for autoimmune inflammation of the brain", Nature, 421:744-748 (2003); Murphy, C.A. et al., "Divergent pro- and anti-inflammatory roles for IL-23 and IL-12 in joint autoimmune inflammation", J. Exp. Med., 198:1951-1957 (2003)).

In human disease, high expression of p40 and p19 has been measured in psoriatic lesions, and Th17 cells have been identified in active lesions in the brain from MS patients and in the gut mucosa of patients with active Crohn's disease (Lee, E. et al., "Increased expression of interleukin 23 p19 and p40 in lesional skin of patients with psoriasis vulgaris", J. Exp. Med., 199:125-130 (2004); Tzartos, J.S. et al., "Interleukin-17 production in central nervous system infiltrating T cells and glial cells is associated with active disease in multiple sclerosis", Am. J. Pathol., 172:146-155 (2008)). The mRNA levels of p19, p40, and p35 in active SLE patients were also shown to be significantly higher compared with those in inactive SLE patients (Huang, X. et al., "Dysregulated expression of interleukin-23 and interleukin-12 subunits in systemic lupus erythematosus patients", Mod. Rheumatol., 17:220-223 (2007)), and T cells from lupus patients have a predominant Th1 phenotype (Tucci, M. et al., "Overexpression of interleukin-12 and T helper 1 predominance in lupus nephritis", Clin. Exp. Immunol., 154:247-254 (2008)).

Moreover, genome-wide association studies have identified a number of loci associated with chronic inflammatory and autoimmune diseases that encode factors that function in the IL-23 and IL-12 pathways. These genes include IL23A, IL12A, IL12B, IL12RB1, IL12RB2, IL23R, JAK2, TYK2, STAT3, and STAT4 (Lees, C.W. et al., "New IBD genetics: common pathways with other diseases", Gut, 60:1739-1753 (2011); Tao, J.H. et al., "Meta-analysis of TYK2 gene polymorphisms association with susceptibility to autoimmune and inflammatory diseases", Mol. Biol. Rep., 38:4663-4672 (2011); Cho, J.H. et al., "Recent insights into the genetics of inflammatory bowel disease", Gastroenterology, 140:1704-1712 (2011)).

Indeed, anti-p40 treatment, which inhibits both IL-12 and IL-23, as well as IL-23-specific anti-p19 therapies have been shown to be efficacious in the treatment of autoimmunity in diseases including psoriasis, Crohn's Disease and psoriatic arthritis (Leonardi, C.L. et al., "PHOENIX 1 study investigators. Efficacy and safety of ustekinumab, a human interleukin-12/23 monoclonal antibody, in patients with psoriasis: 76-week results from a randomized, double-blind, placebo-controlled trial (PHOENIX 1)", Lancet, 371:1665-1674 (2008); Sandborn, W.J. et al., "Ustekinumab Crohn's Disease Study Group. A randomized trial of Ustekinumab, a human interleukin-12/23 monoclonal antibody, in patients with moderate-to-severe Crohn's disease", Gastroenterology, 135:1130-1141 (2008); Gottlieb, A. et al., "Ustekinumab, a human interleukin 12/23 monoclonal antibody, for psoriatic arthritis: randomized, double-blind, placebo-controlled, crossover trial", Lancet, 373:633-640 (2009)). Therefore, agents which inhibit the action of IL-12 and IL-23 may be expected to have therapeutic benefit in human autoimmune disorders.

The Type I group of interferons (IFNs), which include the IFNα members as well as IFNβ, IFNε, IFNκ and IFNω, act through a heterodimer IFNα/β receptor (IFNAR). Type I IFNs have multiple effects in both the innate and adaptive immune systems including activation of both the cellular and humoral immune responses as well as enhancing the expression and release of autoantigens (Hall, J.C. et al., "Type I interferons: crucial participants in disease amplification in autoimmunity", Nat. Rev. Rheumatol., 6:40-49 (2010)).

In patients with systemic lupus erythematosus (SLE), a potentially fatal autoimmune disease, increased serum levels of interferon (IFN)α (a type I interferon) or increased expression of type I IFN-regulated genes (a so-called IFNα signature) in peripheral blood mononuclear cells and in affected organs has been demonstrated in a majority of patients (Bennett, L. et al., "Interferon and granulopoiesis signatures in systemic lupus erythematosus blood", J. Exp. Med., 197:711-723 (2003); Peterson, K.S. et al., "Characterization of heterogeneity in the molecular pathogenesis of lupus nephritis from transcriptional profiles of laser-captured glomeruli", J. Clin. Invest., 113:1722-1733 (2004)), and several studies have shown that serum IFNα levels correlate with both disease activity and severity (Bengtsson, A.A. et al., "Activation of type I interferon system in systemic lupus erythematosus correlates with disease activity but not with antiretroviral antibodies", Lupus, 9:664-671 (2000)). A direct role for IFNα in the pathobiology of lupus is evidenced by the observation that the administration of IFNα to patients with malignant or viral diseases can induce a lupus-like syndrome. Moreover, the deletion of the IFNAR in lupus-prone mice provides high protection from autoimmunity, disease severity and mortality (Santiago-Raber, M.L. et al., "Type-I interferon receptor deficiency reduces lupus-like disease in NZB mice", J. Exp. Med., 197:777-788 (2003)), and genome-wide association studies have identified loci associated with lupus that encode factors that function in the type I interferon pathway, including IRF5, IKBKE, TYK2, and STAT4 (Deng, Y. et al., "Genetic susceptibility to systemic lupus erythematosus in the genomic era", Nat. Rev. Rheumatol., 6:683-692 (2010); Sandling, J.K. et al., "A candidate gene study of the type I interferon pathway implicates IKBKE and IL8 as risk loci for SLE", Eur. J. Hum. Genet., 19:479-484 (2011)). In addition to lupus, there is evidence that aberrant activation of type I interferon-mediated pathways are important in the pathobiology of other autoimmune diseases such as Sjögren's syndrome and scleroderma (Båve, U. et al., "Activation of the type I interferon system in primary Sjögren's syndrome: a possible etiopathogenic mechanism", Arthritis Rheum., 52:1185-1195 (2005); Kim, D. et al., "Induction of interferon-alpha by scleroderma sera containing autoantibodies to topoisomerase I: association of higher interferon-alpha activity with lung fibrosis", Arthritis Rheum., 58:2163-2173 (2008)). Therefore, agents which inhibit the action of type I interferon responses may be expected to have therapeutic benefit in human autoimmune disorders.

Tyrosine kinase 2 (Tyk2) is a member of the Janus kinase (JAK) family of nonreceptor tyrosine kinases and has been shown to be critical in regulating the signal transduction cascade downstream of receptors for IL-12, IL-23 and type I interferons in both mice (Ishizaki, M. et al., "Involvement of Tyrosine Kinase-2 in Both the IL-12/Th1 and IL-23/Th17 Axes In vivo", J. Immunol., 187:181-189 (2011); Prchal-Murphy, M. et al., "TYK2 kinase activity is required for functional type I interferon responses in vivo", PLoS One, 7:e39141 (2012)) and humans (Minegishi, Y. et al., "Human tyrosine kinase 2 deficiency reveals its requisite roles in multiple cytokine signals involved in innate and acquired immunity", Immunity, 25:745-755 (2006)). Tyk2 mediates the receptor-induced phosphorylation of members of the STAT family of transcription factors, an essential signal that leads to the dimerization of STAT proteins and the transcription of STAT-dependent pro-inflammatory genes. Tyk2-deficient mice are resistant to experimental models of colitis, psoriasis and multiple sclerosis, demonstrating the importance of Tyk2-mediated signaling in autoimmunity and related disorders (Ishizaki, M. et al., "Involvement of Tyrosine Kinase-2 in Both the IL-12/Th1 and IL-23/Th17 Axes In vivo", J. Immunol., 187:181-189 (2011); Oyamada, A. et al., "Tyrosine kinase 2 plays critical roles in the pathogenic CD4 T cell responses for the development of experimental autoimmune encephalomyelitis", J. Immunol., 183:7539-7546 (2009)).

In humans, individuals expressing an inactive variant of Tyk2 are protected from multiple sclerosis and possibly other autoimmune disorders (Couturier, N. et al., "Tyrosine kinase 2 variant influences T lymphocyte polarization and multiple sclerosis susceptibility", Brain, 134:693-703 (2011)). Genome-wide association studies have shown other variants of Tyk2 to be associated with autoimmune disorders such as Crohn's Disease, psoriasis, systemic lupus erythematosus, and rheumatoid arthritis, further demonstrating the importance of Tyk2 in autoimmunity (Ellinghaus, D. et al., "Combined Analysis of Genome-wide Association Studies for Crohn Disease and Psoriasis Identifies Seven Shared Susceptibility Loci", Am. J. Hum. Genet., 90:636-647 (2012); Graham, D. et al., "Association of polymorphisms across the tyrosine kinase gene, TYK2 in UK SLE families", Rheumatology (Oxford), 46:927-930 (2007); Eyre, S. et al., "High-density genetic mapping identifies new susceptibility loci for rheumatoid arthritis", Nat. Genet., 44:1336-1340 (2012)).

WO2014/074661 A1; WO2015/069310 A1; WO2018/071794 A1; WO2020/086616 A1 and WO2020/156311 A1 describe compounds for the inhibition of Tyk-2.

In view of the conditions that may benefit by treatment involving the modulation of cytokines and/or interferons, new compounds capable of modulating cytokines and/or interferons, such as IL-12, IL-23 and/or IFNα, and methods of using these compounds may provide substantial therapeutic benefits to a wide variety of patients in need thereof.

### SUMMARY OF THE INVENTION

The invention is directed to compounds of Formula I, which are useful as modulators of IL-12, IL-23 and/or IFNα by inhibiting Tyk2-mediated signal transduction.

The present invention also provides pharmaceutical compositions comprising a pharmaceutically acceptable carrier and at least one of the compounds of the present invention.

The present invention also provides the compounds of the present invention for use in a method for the modulation of IL-12, IL-23 and/or IFNα by inhibiting Tyk-2-mediated signal transduction.

The present invention also provides the compounds of the present invention for use in a method for treating proliferative, metabolic, allergic, autoimmune and inflammatory diseases.

A preferred embodiment is the compounds of the present invention for use in a method for treating inflammatory and autoimmune diseases or diseases. For the purposes of this invention, an inflammatory and autoimmune disease or disorder includes any disease having an inflammatory or autoimmune component.

The present invention also provides the use of the compounds of the present invention for the manufacture of a medicament for the treatment of cancers.

The present invention also provides the compounds of the present invention for use in therapy.

These and other features of the invention will be set forth in the expanded form as the disclosure continues.

### DETAILED DESCRIPTION OF THE EMBODIMENTS OF THE INVENTION

In a first aspect of the present invention, there is provided a compound of formula (I) 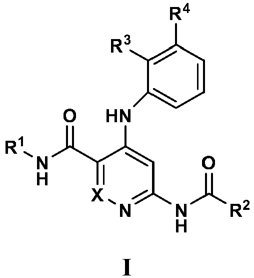 wherein
X is N or CH;
R¹ is H, CD₃, C₁₋₃ alkyl or C₃₋₆ cycloalkyl;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 1 or 2;
r is 0, 1, 2, 3, 4 or 5;
or a stereoisomer or pharmaceutically acceptable salt thereof.

In a second aspect of the present invention, there is provided a compound of the 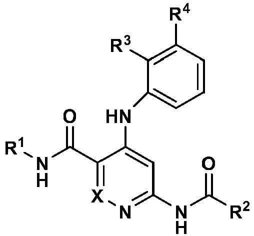 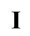 wherein
X is N or CH;
R¹ is H or CD₃;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 1 or 2;
r is 0, 1, 2, 3, 4 or 5;
or a stereoisomer or pharmaceutically acceptable salt thereof.

In a third aspect of the present invention, there is provided a compound of the formula 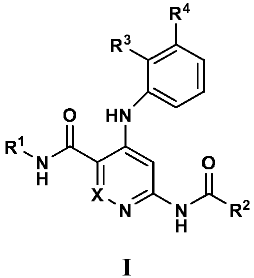 wherein
X is N or CH;
R¹ is H or CD₃;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1, 2, 3, 4 or 5;
or a stereoisomer or pharmaceutically acceptable salt thereof.

In a 4th aspect of the present invention, there is provided a compound of the formula 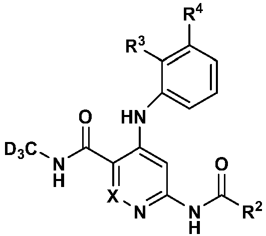 wherein
X is N or CH;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1, 2, 3, 4 or 5;
or a stereoisomer or pharmaceutically acceptable salt thereof.

In a 5th aspect of the present invention, there is provided a compound of the formula 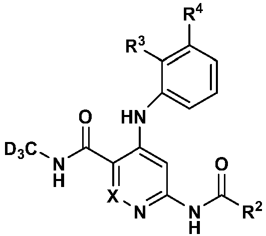 wherein
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1 or 2;
or a stereoisomer or pharmaceutically acceptable salt thereof.

In a 6th aspect of the present invention, there is provided a compound of the formula 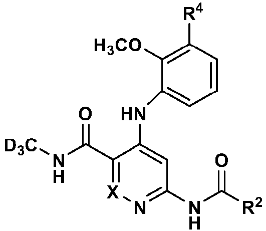 wherein
R² is C₁₋₆ alkoxy;
R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1 or 2;
or a stereoisomer or pharmaceutically acceptable salt thereof.

In a 7th aspect of the present invention, there is provided a compound of the formula 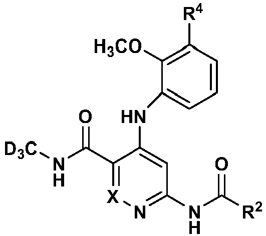 wherein
R² is C₁₋₆ alkoxy;
R⁴ is absent or a triazole, thiazole, tetrazole or oxadiazole group each of which is substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1 or 2;
or a stereoisomer or pharmaceutically acceptable salt thereof.

In an 8th aspect of the present invention, there is provided a compound of the formula 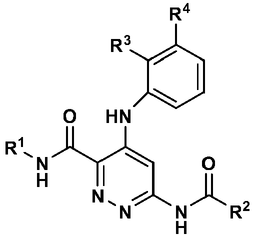 wherein
R¹ is H, CD₃, C₁₋₃ alkyl or C₃₋₆ cycloalkyl;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 1 or 2;
r is 0, 1, 2, 3, 4 or 5;
or a stereoisomer or pharmaceutically acceptable salt thereof.

In a 9th aspect of the present invention, there is provided a compound of the formula 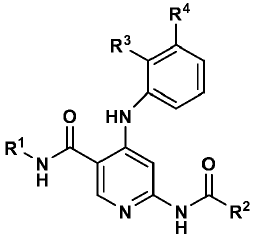 wherein
R¹ is H, CD₃, C₁₋₃ alkyl or C₃₋₆ cycloalkyl;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 1 or 2;
r is 0, 1, 2, 3, 4 or 5;
or a stereoisomer or pharmaceutically acceptable salt thereof.

In another aspect, there is provided a compound selected from the exemplified examples within the scope of the first aspect, or a pharmaceutically acceptable salt or stereoisomer thereof.

In another aspect, there is provided a compound selected from any subset list of compounds within the scope of any of the above aspects.

In another embodiment, there is provided a pharmaceutical composition comprising one or more compounds of formula I and a pharmaceutically acceptable carrier or diluent.

The present invention is also directed to pharmaceutical compositions useful in treating diseases associated with the modulation of IL-12, IL-23 and/or IFNα by acting on Tyk-2 to cause signal transduction inhibition, comprising compounds of formula I, or pharmaceutically-acceptable salts thereof, and pharmaceutically-acceptable carriers or diluents.

The invention further relates to the compounds of the present invention for use in methods of treating diseases associated with the modulation of IL-12, IL-23, and/or IFNα.

The present invention also provides processes and intermediates for making the compounds of the present invention.

The present invention also provides the compounds of the present invention for use in a method for treating proliferative, metabolic, allergic, autoimmune and inflammatory diseases (or use of the compounds of the present invention for the manufacture of a medicament for the treatment of these diseases).

The present invention also provides the compounds of the present invention for use in a method of treating an inflammatory or autoimmune disease (or use of the compounds of the present invention for the manufacture of a medicament for the treatment of these diseases).

The present invention also provides the compounds of the present invention for use in a method for treating a disease (or use of the compounds of the present invention for the manufacture of a medicament for the treatment of these diseases), wherein the disease is rheumatoid arthritis, multiple sclerosis, systemic lupus erythematosus (SLE), lupus nephritis, cutaneous lupus, inflammatory bowel disease, psoriasis, Crohn's Disease, psoriatic arthritis, Sjögren's syndrome, systemic scleroderma, ulcerative colitis, Graves' disease, discoid lupus erythematosus, adult onset Stills, systemic onset juvenile idiopathic arthritis, gout, gouty arthritis, type 1 diabetes, insulin dependent diabetes mellitus, sepsis, septic shock, Shigellosis, pancreatitis (acute or chronic), glomerulonephritis, autoimmune gastritis, diabetes, autoimmune hemolytic anemia, autoimmune neutropenia, thrombocytopenia, atopic dermatitis, myasthenia gravis, pancreatitis (acute or chronic), ankylosing spondylitis, pemphigus vulgaris, Goodpasture's disease, antiphospholipid syndrome, idiopathic thrombocytopenia, ANCA-associated vasculitis, pemphigus, Kawasaki disease, Chronic Inflammatory Demyelinating Polyneuropathy (CIDP), dermatomyositis, polymyositis, uveitis, Guillain-Barré syndrome, autoimmune pulmonary inflammation, autoimmune thyroiditis, autoimmune inflammatory eye disease, and chronic demyelinating polyneuropathy.

The present invention also provides the compounds of the present invention for use in a method of treating an inflammatory or autoimmune disease (or use of the compounds of the present invention for the manufacture of a medicament for the treatment of said diseases), wherein the disease is selected from systemic lupus erythematosus (SLE), lupus nephritis, cutaneous lupus, Crohn's Disease, ulcerative colitis, type 1 diabetes, psoriasis, rheumatoid arthritis, systemic onset juvenile idiopathic arthritis, ankylosing spondylitis, and multiple sclerosis.

The present invention also provides the compounds of the present invention for use in a method for treating rheumatoid arthritis or the use of the compounds of the present invention for the manufacture of a medicament for the treatment of rheumatoid arthritis.

In addition, the present invention also provides the compounds of the present invention for use in a method of treating a condition (or use of the compounds of the present invention for the manufacture of a medicament for the treatment of these conditions), wherein the condition is selected from acute myelogenous leukemia, chronic myelogenous leukemia, metastatic melanoma, Kaposi's sarcoma, multiple myeloma, solid tumors, ocular neovascularization, and infantile haemangiomas, B cell lymphoma, systemic lupus erythematosus (SLE), rheumatoid arthritis, psoriatic arthritis, multiple vasculitides, idiopathic thrombocytopenic purpura (ITP), myasthenia gravis, allergic rhinitis, multiple sclerosis (MS), transplant rejection, Type I diabetes, membranous nephritis, inflammatory bowel disease, autoimmune hemolytic anemia, autoimmune thyroiditis, cold and warm agglutinin diseases, Evans syndrome, hemolytic uremic syndrome/thrombotic thrombocytopenic purpura (HUS/TTP), sarcoidosis, Sjögren's syndrome, peripheral neuropathies, pemphigus vulgaris and asthma.

The present invention also provides the compounds of the present invention for use in a method of treating an IL-12, IL-23, and/or IFNα mediated disease (or use of the compounds of the present invention for the manufacture of a medicament for the treatment of these diseases).

The present invention also provides the compounds of the present invention for use in a method of treating an IL-12, IL-23 and/or IFNα mediated disease (or use of the compounds of the present invention for the manufacture of a medicament for the treatment of these diseases) , wherein the IL-12, IL-23 and/or IFNα mediated disease is a disease modulated by IL-12, IL-23 and/or IFNα.

The present invention also provides the compounds of the present invention for use in a method of treating diseases, in combination with other therapeutic agents.

The present invention also provides the compounds of the present invention for use in therapy.

In another embodiment, compounds of formula I are selected from exemplified compounds or combinations of exemplified compounds or other embodiments herein.

In another embodiment are compounds having an IC₅₀ < 1000 nM in at least one of the assays described below.

The present invention may be embodied in other specific forms. It is understood that any and all embodiments of the present invention may be taken in conjunction with any other embodiment or embodiments to describe additional more preferred embodiments. It is also to be understood that each individual element of the preferred embodiments is its own independent preferred embodiment. Furthermore, any element of an embodiment is meant to be combined with any and all other elements from any embodiment to describe an additional embodiment.

### DETAILED DESCRIPTION OF THE INVENTION

The following are definitions of terms used in this specification and appended claims. The initial definition provided for a group or term herein applies to that group or term throughout the specification and claims, individually or as part of another group, unless otherwise indicated.

Compounds of this invention may have one or more asymmetric centers. Unless otherwise indicated, all chiral (enantiomeric and diastereomeric) and racemic forms of compounds of the present invention are included in the present invention. Many geometric isomers of olefins, C=N double bonds, and the like can also be present in the compounds, and all such stable isomers are contemplated in the present invention. *Cis-* and *trans-*geometric isomers of the compounds of the present invention are described and may be isolated as a mixture of isomers or as separated isomeric forms. The present compounds can be isolated in optically active or racemic forms. It is well known in the art how to prepare optically active forms, such as by resolution of racemic forms or by synthesis from optically active starting materials. All chiral, (enantiomeric and diastereomeric) and racemic forms and all geometric isomeric forms of a structure are intended, unless the specific stereochemistry or isomer form is specifically indicated.

When any variable (*e.g.,* R³) occurs more than one time in any constituent or formula for a compound, its definition at each occurrence is independent of its definition at every other occurrence. Thus, for example, if a group is shown to be substituted with 0-2 R³, then said group may optionally be substituted with up to two R³ groups and R³ at each occurrence is selected independently from the definition of R³. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

In cases wherein there are nitrogen atoms (*e.g.*, amines) on compounds of the present invention, these can be converted to N-oxides by treatment with an oxidizing agent (*e.g.,* MCPBA and/or hydrogen peroxides) to afford other compounds of this invention. Thus, all shown and claimed nitrogen atoms are considered to cover both the shown nitrogen and its N-oxide (N→O) derivative.

In accordance with a convention used in the art, 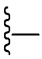 is used in structural formulas herein to depict the bond that is the point of attachment of the moiety or substituent to the core or backbone structure.

A dash "-" that is not between two letters or symbols is used to indicate a point of attachment for a substituent. For example, -CONH₂ is attached through the carbon atom.

The term "optionally substituted" in reference to a particular moiety of the compound of Formula I (*e.g.,* an optionally substituted heteroaryl group) refers to a moiety having 0, 1, 2, or more substituents. For example, "optionally substituted alkyl" encompasses both "alkyl" and "substituted alkyl" as defined below. It will be understood by those skilled in the art, with respect to any group containing one or more substituents, that such groups are not intended to introduce any substitution or substitution patterns that are sterically impractical, synthetically non-feasible and/or inherently unstable.

As used herein, the term "at least one chemical entity" is interchangeable with the term "a compound".

As used herein, the term "alkyl" or "alkylene" is intended to include both branched and straight-chain saturated aliphatic hydrocarbon groups having the specified number of carbon atoms. For example, "C₁₋₁₀ alkyl" (or alkylene), is intended to include C₁, C₂, C₃, C₄, C₅, C₆, C₇, C₈, C₉, and C₁₀ alkyl groups. Additionally, for example, "C₁-C₆ alkyl" denotes alkyl having 1 to 6 carbon atoms. Alkyl groups can be unsubstituted or substituted so that one or more of its hydrogens are replaced by another chemical group. Example alkyl groups include, but are not limited to, methyl (Me), ethyl (Et), propyl (*e.g.*, n-propyl and isopropyl), butyl (*e.g.,* n-butyl, isobutyl, t-butyl), pentyl (*e.g.,* n-pentyl, isopentyl, neopentyl), and the like.

"Alkenyl" or "alkenylene" is intended to include hydrocarbon chains of either straight or branched configuration and having one or more double carbon-carbon bonds that may occur in any stable point along the chain. For example, "C₂₋₆ alkenyl" (or alkenylene), is intended to include C₂, C₃, C₄, C₅, and C₆ alkenyl groups. Examples of alkenyl include, but are not limited to, ethenyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl, 4-methyl-3-pentenyl, and the like.

"Alkynyl" or "alkynylene" is intended to include hydrocarbon chains of either straight or branched configuration and having one or more triple carbon-carbon bonds that may occur in any stable point along the chain. For example, "C₂₋₆ alkynyl" (or alkynylene), is intended to include C₂, C₃, C₄, C₅, and C₆ alkynyl groups; such as ethynyl, propynyl, butynyl, pentynyl, hexynyl and the like.

One skilled in the field will understand that, when the designation "CO₂" is used herein, this is intended to refer to the group

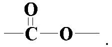

When the term "alkyl" is used together with another group, such as in "arylalkyl", this conjunction defines with more specificity at least one of the substituents that the substituted alkyl will contain. For example, "arylalkyl" refers to a substituted alkyl group as defined above where at least one of the substituents is an aryl, such as benzyl. Thus, the term aryl(C₀₋₄)alkyl includes a substituted lower alkyl having at least one aryl substituent and also includes an aryl directly bonded to another group, *i.e.,* aryl(C₀)alkyl. The term "heteroarylalkyl" refers to a substituted alkyl group as defined above where at least one of the substituents is a heteroaryl.

When reference is made to a substituted alkenyl, alkynyl, alkylene, alkenylene, or alkynylene group, these groups are substituted with one to three substituents as defined above for substituted alkyl groups.

The term "alkoxy" refers to an oxygen atom substituted by alkyl or substituted alkyl, as defined herein. For example, the term "alkoxy" includes the group -O-C₁₋₆alkyl such as methoxy, ethoxy, propoxy, isopropoxy, n-butoxy, sec-butoxy, tert-butoxy, pentoxy, 2-pentyloxy, isopentoxy, neopentoxy, hexoxy, 2-hexoxy, 3-hexoxy, 3-methylpentoxy, and the like. "Lower alkoxy" refers to alkoxy groups having one to four carbons.

It should be understood that the selections for all groups, including for example, alkoxy, thioalkyl, and aminoalkyl, will be made by one skilled in the field to provide stable compounds.

The term "substituted", as used herein, means that any one or more hydrogens on the designated atom or group is replaced with a selection from the indicated group, provided that the designated atom's normal valence is not exceeded. When a substituent is oxo, or keto, (*i.e.,* =O) then 2 hydrogens on the atom are replaced. Keto substituents are not present on aromatic moieties. Unless otherwise specified, substituents are named into the core structure. For example, it is to be understood that when (cycloalkyl)alkyl is listed as a possible substituent, the point of attachment of this substituent to the core structure is in the alkyl portion. Ring double bonds, as used herein, are double bonds that are formed between two adjacent ring atoms (*e.g.,* C=C, C=N, or N=N).

Combinations of substituents and/or variables are permissible only if such combinations result in stable compounds or useful synthetic intermediates. A stable compound or stable structure is meant to imply a compound that is sufficiently robust to survive isolation from a reaction mixture to a useful degree of purity, and subsequent formulation into an efficacious therapeutic agent. It is preferred that the presently recited compounds do not contain a N-halo, S(O)₂H, or S(O)H group.

The term "cycloalkyl" refers to cyclized alkyl groups, including mono-, bi- or polycyclic ring systems. C₃₋₇ cycloalkyl is intended to include C₃, C₄, C₅, C₆, and C₇ cycloalkyl groups. Example cycloalkyl groups include, but are not limited to, cyclopropyl, cyclobutyl, cyclopentyl, cyclohexyl, norbornyl, and the like. As used herein, "carbocycle" or "carbocyclic residue" is intended to mean any stable 3-, 4-, 5-, 6-, or 7-membered monocyclic or bicyclic or 7-, 8-, 9-, 10-, 11-, 12-, or 13-membered bicyclic or tricyclic ring, any of which may be saturated, partially unsaturated, unsaturated or aromatic. Examples of such carbocycles include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, [3.3.0]bicyclooctane, [4.3.0]bicyclononane, [4.4.0]bicyclodecane, [2.2.2]bicyclooctane, fluorenyl, phenyl, naphthyl, indanyl, adamantyl, anthracenyl, and tetrahydronaphthyl (tetralin). As shown above, bridged rings are also included in the definition of carbocycle (*e.g.*, [2.2.2]bicyclooctane). Preferred carbocycles, unless otherwise specified, are cyclopropyl, cyclobutyl, cyclopentyl, cyclohexyl, and phenyl. When the term "carbocycle" is used, it is intended to include "aryl". A bridged ring occurs when one or more carbon atoms link two non-adjacent carbon atoms. Preferred bridges are one or two carbon atoms. It is noted that a bridge always converts a monocyclic ring into a bicyclic ring. When a ring is bridged, the substituents recited for the ring may also be present on the bridge.

The term "aryl" refers to monocyclic or bicyclic aromatic hydrocarbon groups having 6 to 12 carbon atoms in the ring portion, such as phenyl, and naphthyl groups, each of which may be substituted.

Accordingly, in compounds of formula I, the term "cycloalkyl" includes cyclopropyl, cyclobutyl, cyclopentyl, cyclohexyl, cycloheptyl, bicyclooctyl, etc

The term "halo" or "halogen" refers to chloro, bromo, fluoro and iodo.

The term "haloalkyl" means a substituted alkyl having one or more halo substituents. For example, "haloalkyl" includes mono, bi, and trifluoromethyl.

The term "haloalkoxy" means an alkoxy group having one or more halo substituents. For example, "haloalkoxy" includes OCF₃.

The terms "heterocycle", "heterocycloalkyl", "heterocyclo", "heterocyclic", or "heterocyclyl" may be used interchangeably and refer to substituted and unsubstituted 3-to 7-membered monocyclic groups, 7- to 11-membered bicyclic groups, and 10- to 15-membered tricyclic groups, in which at least one of the rings has at least one heteroatom (O, S or N), said heteroatom containing ring preferably having 1, 2, or 3 heteroatoms selected from O, S, and N. Each ring of such a group containing a heteroatom can contain one or two oxygen or sulfur atoms and/or from one to four nitrogen atoms provided that the total number of heteroatoms in each ring is four or less, and further provided that the ring contains at least one carbon atom. The nitrogen and sulfur atoms may optionally be oxidized and the nitrogen atoms may optionally be quaternized. The fused rings completing the bicyclic and tricyclic groups may contain only carbon atoms and may be saturated, partially saturated, or fully unsaturated. The heterocyclo group may be attached at any available nitrogen or carbon atom. As used herein the terms "heterocycle", "heterocycloalkyl", "heterocyclo", "heterocyclic", and "heterocyclyl" include "heteroaryl" groups, as defined below.

In addition to the heteroaryl groups described below, exemplary monocyclic heterocyclyl groups include azetidinyl, pyrrolidinyl, oxetanyl, imidazolinyl, oxazolidinyl, isoxazolinyl, thiazolidinyl, isothiazolidinyl, tetrahydrofuranyl, piperidyl, piperazinyl, 2-oxopiperazinyl, 2-oxopiperidyl, 2-oxopyrrolodinyl, 2-oxoazepinyl, azepinyl, 1-pyridonyl, 4-piperidonyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl and the like. Exemplary bicyclic heterocyclo groups include quinuclidinyl.

The term "heteroaryl" refers to substituted and unsubstituted aromatic 5- or 6-membered monocyclic groups, 9- or 10-membered bicyclic groups, and 11- to 14-membered tricyclic groups which have at least one heteroatom (O, S or N) in at least one of the rings, said heteroatom-containing ring preferably having 1, 2, or 3 heteroatoms selected from O, S, and N. Each ring of the heteroaryl group containing a heteroatom can contain one or two oxygen or sulfur atoms and/or from one to four nitrogen atoms provided that the total number of heteroatoms in each ring is four or less and each ring has at least one carbon atom. The fused rings completing the bicyclic and tricyclic groups may contain only carbon atoms and may be saturated, partially saturated, or unsaturated. The nitrogen and sulfur atoms may optionally be oxidized and the nitrogen atoms may optionally be quaternized. Heteroaryl groups which are bicyclic or tricyclic must include at least one fully aromatic ring but the other fused ring or rings may be aromatic or nonaromatic. The heteroaryl group may be attached at any available nitrogen or carbon atom of any ring. As valence allows, if said further ring is cycloalkyl or heterocyclo it is additionally optionally substituted with =O (oxo).

Exemplary monocyclic heteroaryl groups include pyrrolyl, pyrazolyl, pyrazolinyl, imidazolyl, oxazolyl, isoxazolyl, thiazolyl, thiadiazolyl, isothiazolyl, furanyl, thienyl, oxadiazolyl, pyridyl, pyrazinyl, pyrimidinyl, pyridazinyl, triazinyl and the like.

Exemplary bicyclic heteroaryl groups include indolyl, benzothiazolyl, benzodioxolyl, benzoxazolyl, benzothienyl, quinolinyl, tetrahydroisoquinolinyl, isoquinolinyl, benzimidazolyl, benzopyranyl, indolizinyl, benzofuranyl, chromonyl, coumarinyl, benzopyranyl, cinnolinyl, quinoxalinyl, indazolyl, pyrrolopyridyl, furopyridyl, dihydroisoindolyl, tetrahydroquinolinyl and the like.

Exemplary tricyclic heteroaryl groups include carbazolyl, benzindolyl, phenanthrollinyl, acridinyl, phenanthridinyl, xanthenyl and the like.

In compounds of formula I, preferred heteroaryl groups include: 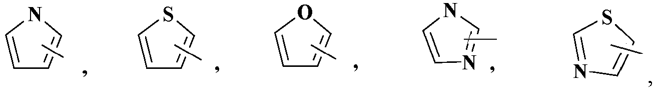 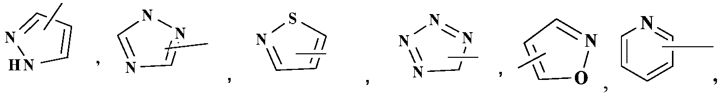 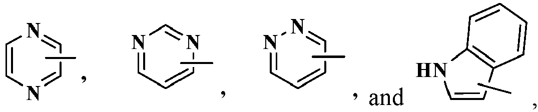 and the like, which optionally may be substituted at any available carbon or nitrogen atom.

Unless otherwise indicated, when reference is made to a specifically-named aryl (*e.g.,* phenyl), cycloalkyl (*e.g.,* cyclohexyl), heterocyclo (*e.g.,* pyrrolidinyl, piperidinyl, and morpholinyl) or heteroaryl (*e.g.*, tetrazolyl, imidazolyl, pyrazolyl, triazolyl, thiazolyl, and furyl) the reference is intended to include rings having 0 to 3, preferably 0 to 2, substituents selected from those recited above for the aryl, cycloalkyl, heterocyclo and/or heteroaryl groups, as appropriate.

The term "carbocyclyl" or "carbocyclic" refers to a saturated or unsaturated monocyclic or bicyclic ring in which all atoms of all rings are carbon. Thus, the term includes cycloalkyl and aryl rings. Monocyclic carbocycles have 3 to 6 ring atoms, still more typically 5 or 6 ring atoms. Bicyclic carbocycles have 7 to 12 ring atoms, *e.g.,* arranged as a bicyclo [4,5], [5,5], [5,6] or [6,6] system, or 9 or 10 ring atoms arranged as a bicyclo [5,6] or [6,6] system. Examples of mono- and bicyclic carbocycles include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, phenyl and naphthyl. The carbocyclic ring may be substituted in which case the substituents are selected from those recited above for cycloalkyl and aryl groups.

The term "heteroatoms" shall include oxygen, sulfur and nitrogen.

When the term "unsaturated" is used herein to refer to a ring or group, the ring or group may be fully unsaturated or partially unsaturated.

Throughout the specification, groups and substituents thereof may be chosen by one skilled in the field to provide stable moieties and compounds useful as pharmaceutically-acceptable compounds and/or intermediate compounds useful in making pharmaceutically-acceptable compounds.

The compounds of formula I may exist in a free form (with no ionization) or can form salts which are also within the scope of this invention. Unless otherwise indicated, reference to an inventive compound is understood to include reference to the free form and to salts thereof. The term "salt(s)" denotes acidic and/or basic salts formed with inorganic and/or organic acids and bases. In addition, the term "salt(s)" may include zwitterions (inner salts), *e.g.,* when a compound of formula I, contains both a basic moiety, such as an amine or a pyridine or imidazole ring, and an acidic moiety, such as a carboxylic acid. Pharmaceutically acceptable (*i.e.,* non-toxic, physiologically acceptable) salts are preferred, such as, for example, acceptable metal and amine salts in which the cation does not contribute significantly to the toxicity or biological activity of the salt. However, other salts may be useful, *e.g.,* in isolation or purification steps which may be employed during preparation, and thus, are contemplated within the scope of the invention. Salts of the compounds of the formula I may be formed, for example, by reacting a compound of the formula I with an amount of acid or base, such as an equivalent amount, in a medium such as one in which the salt precipitates or in an aqueous medium followed by lyophilization.

Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, 2-hydroxyethanesulfonates, lactates, maleates (formed with maleic acid), methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

Exemplary basic salts include ammonium salts, alkali metal salts such as sodium, lithium, and potassium salts; alkaline earth metal salts such as calcium and magnesium salts; barium, zinc, and aluminum salts; salts with organic bases (for example, organic amines) such as trialkylamines such as triethylamine, procaine, dibenzylamine, N-benzyl-β-phenethylamine, 1-ephenamine, *N,N*'-dibenzylethylene-diamine, dehydroabietylamine, N-ethylpiperidine, benzylamine, dicyclohexylamine or similar pharmaceutically acceptable amines and salts with amino acids such as arginine, lysine and the like. Basic nitrogen-containing groups may be quaternized with agents such as lower alkyl halides (*e.g.*, methyl, ethyl, propyl, and butyl chlorides, bromides and iodides), dialkyl sulfates (*e.g.,* dimethyl, diethyl, dibutyl, and diamyl sulfates), long chain halides (*e.g.,* decyl, lauryl, myristyl and stearyl chlorides, bromides and iodides), aralkyl halides (*e.g.*, benzyl and phenethyl bromides), and others. Preferred salts include monohydrochloride, hydrogensulfate, methanesulfonate, phosphate or nitrate salts.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically-acceptable salts" refer to derivatives of the disclosed compounds wherein the parent compound is modified by making acid or base salts thereof. Examples of pharmaceutically-acceptable salts include, but are not limited to, mineral or organic acid salts of basic groups such as amines; and alkali or organic salts of acidic groups such as carboxylic acids. The pharmaceutically-acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic, and the like.

The pharmaceutically-acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 18th Edition, Mack Publishing Company, Easton, PA (1990).

All stereoisomers of the compounds of the instant invention are contemplated, either in admixture or in pure or substantially pure form. Stereoisomers may include compounds which are optical isomers through possession of one or more chiral atoms, as well as compounds which are optical isomers by virtue of limited rotation about one or more bonds (atropisomers). The definition of compounds according to the invention embraces all the possible stereoisomers and their mixtures. It very particularly embraces the racemic forms and the isolated optical isomers having the specified activity. The racemic forms can be resolved by physical methods, such as, for example, fractional crystallization, separation or crystallization of diastereomeric derivatives or separation by chiral column chromatography. The individual optical isomers can be obtained from the racemates from the conventional methods, such as, for example, salt formation with an optically active acid followed by crystallization.

The present invention is intended to include all isotopes of atoms occurring in the present compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Prodrugs and solvates of the inventive compounds are also contemplated. The term "prodrug" denotes a compound which, upon administration to a subject, undergoes chemical conversion by metabolic or chemical processes to yield a compound of the formula I, and/or a salt and/or solvate thereof. Any compound that will be converted *in vivo* to provide the bioactive agent (*i.e.,* the compound for formula I) is a prodrug. For example, compounds containing a carboxy group can form physiologically hydrolyzable esters which serve as prodrugs by being hydrolyzed in the body to yield formula I compounds *per se.* Such prodrugs are preferably administered orally since hydrolysis in many instances occurs principally under the influence of the digestive enzymes. Parenteral administration may be used where the ester *per se* is active, or in those instances where hydrolysis occurs in the blood. Examples of physiologically hydrolyzable esters of compounds of formula I include C₁₋₆alkylbenzyl, 4-methoxybenzyl, indanyl, phthalyl, methoxymethyl, C₁₋₆alkanoyloxy-C₁₋₆alkyl, *e.g.,* acetoxymethyl, pivaloyloxymethyl or propionyloxymethyl, C₁₋₆alkoxycarbonyloxy-C₁₋₆alkyl, *e.g.,* methoxycarbonyl-oxymethyl or ethoxycarbonyloxymethyl, glycyloxymethyl, phenylglycyloxymethyl, (5-methyl-2-oxo-1,3-dioxolen-4-yl)-methyl and other well known physiologically hydrolyzable esters used, for example, in the penicillin and cephalosporin arts. Such esters may be prepared by conventional techniques known in the art.

Various forms of prodrugs are well known in the art and are described in Rautio, J. et al., Nature Review Drug Discovery, 17, 559-587 (2018).

Compounds of the formula I and salts thereof may exist in their tautomeric form, in which hydrogen atoms are transposed to other parts of the molecules and the chemical bonds between the atoms of the molecules are consequently rearranged. It should be understood that all tautomeric forms, insofar as they may exist, are included within the invention. Additionally, inventive compounds may have *trans-* and *cis*-isomers.

It should further be understood that solvates (*e.g.*, hydrates) of the compounds of Formula I are also with the scope of the present invention. Methods of solvation are generally known in the art.

### UTILITY

The compounds of the invention modulate IL-23-stimulated and IFNα-stimulated cellular functions, including gene transcription. Other types of cellular functions that may be modulated by the compounds of the instant invention include, but are not limited to, IL-12-stimulated responses.

Accordingly, compounds of formula I have utility in treating conditions associated with the modulation of the function of IL-23 or IFNα, and particularly the selective inhibition of function of IL-23, IL-12 and/or IFNα, by acting on Tyk2 to mediate signal transduction. Such conditions include IL-23-, IL-12-, or IFNα-associated diseases in which pathogenic mechanisms are mediated by these cytokines.

As used herein, the terms "treating" or "treatment" encompass the treatment of a disease state in a mammal, particularly in a human, and include: (a) preventing or delaying the occurrence of the disease state in a mammal, in particular, when such mammal is predisposed to the disease state but has not yet been diagnosed as having it; (b) inhibiting the disease state, *i.e.,* arresting its development; and/or (c) achieving a full or partial reduction of the symptoms or disease state, and/or alleviating, ameliorating, lessening, or curing the disease or disorder and/or its symptoms.

In view of their activity as modulators of IL-23-, IL-12 and IFNα-stimulated cellular responses, compounds of Formula I are useful in treating IL-23-, IL-12- or IFNα-associated diseases including, but not limited to, inflammatory diseases such as Crohn's disease, ulcerative colitis, asthma, graft versus host disease, allograft rejection, chronic obstructive pulmonary disease; autoimmune diseases such as Graves' disease, rheumatoid arthritis, systemic lupus erythematosis, cutaneous lupus, lupus nephritis, discoid lupus erythematosus, psoriasis; auto-inflammatory diseases including CAPS, TRAPS, FMF, adult onset stills, systemic onset juvenile idiopathic arthritis, gout, gouty arthritis; metabolic diseases including type 2 diabetes, atherosclerosis, myocardial infarction; destructive bone disorders such as bone resorption disease, osteoarthritis, osteoporosis, multiple myeloma-related bone disorder; proliferative disorders such as acute myelogenous leukemia, chronic myelogenous leukemia; angiogenic disorders such as angiogenic disorders including solid tumors, ocular neovascularization, and infantile haemangiomas; infectious diseases such as sepsis, septic shock, and Shigellosis; neurodegenerative diseases such as Alzheimer's disease, Parkinson's disease, cerebral ischemias or neurodegenerative disease caused by traumatic injury, oncologic and viral diseases such as metastatic melanoma, Kaposi's sarcoma, multiple myeloma, and HIV infection and CMV retinitis, AIDS, respectively.

More particularly, the specific conditions or diseases that may be treated with the inventive compounds include, without limitation, pancreatitis (acute or chronic), asthma, allergies, adult respiratory distress syndrome, chronic obstructive pulmonary disease, glomerulonephritis, rheumatoid arthritis, systemic lupus erythematosis, cutaneous lupus, lupus nephritis, discoid lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, graft vs. host disease, inflammatory reaction induced by endotoxin, tuberculosis, atherosclerosis, muscle degeneration, cachexia, psoriatic arthritis, Reiter's syndrome, gout, traumatic arthritis, rubella arthritis, acute synovitis, pancreatic β-cell disease; diseases characterized by massive neutrophil infiltration; rheumatoid spondylitis, gouty arthritis and other arthritic conditions, cerebral malaria, chronic pulmonary inflammatory disease, silicosis, pulmonary sarcoidosis, bone resorption disease, allograft rejections, fever and myalgias due to infection, cachexia secondary to infection, keloid formation, scar tissue formation, ulcerative colitis, pyresis, influenza, osteoporosis, osteoarthritis, acute myelogenous leukemia, chronic myelogenous leukemia, metastatic melanoma, Kaposi's sarcoma, multiple myeloma, sepsis, septic shock, and Shigellosis; Alzheimer's disease, Parkinson's disease, cerebral ischemias or neurodegenerative disease caused by traumatic injury; angiogenic disorders including solid tumors, ocular neovascularization, and infantile haemangiomas; viral diseases including acute hepatitis infection (including hepatitis A, hepatitis B and hepatitis C), HIV infection and CMV retinitis, AIDS, ARC or malignancy, and herpes; stroke, myocardial ischemia, ischemia in stroke heart attacks, organ hypoxia, vascular hyperplasia, cardiac and renal reperfusion injury, thrombosis, cardiac hypertrophy, thrombin-induced platelet aggregation, endotoxemia and/or toxic shock syndrome, conditions associated with prostaglandin endoperoxidase syndase-2, and pemphigus vulgaris. Preferred methods of treatment are those wherein the condition is selected from Crohn's disease, ulcerative colitis, allograft rejection, rheumatoid arthritis, psoriasis, ankylosing spondylitis, psoriatic arthritis, and pemphigus vulgaris. Alternatively preferred methods of treatment are those wherein the condition is selected from ischemia reperfusion injury, including cerebral ischemia reperfusions injury arising from stroke and cardiac ischemia reperfusion injury arising from myocardial infarction. Another preferred method of treatment is one in which the condition is multiple myeloma.

When the terms "IL-23-, IL-12- and/or IFNα-associated condition" or "IL-23-, IL-12- and/or IFNα-associated disease or disorder" are used herein, each is intended to encompass all of the conditions identified above as if repeated at length, as well as any other condition that is affected by IL-23, IL-12 and/or IFNα.

The present invention thus provides methods for treating such conditions, comprising administering to a subject in need thereof a therapeutically-effective amount of at least one compound of Formula I or a salt thereof. "Therapeutically effective amount" is intended to include an amount of a compound of the present invention that is effective when administered alone or in combination to inhibit IL-23, IL-12 and/or IFNα function and/or treat diseases.

The methods of treating IL-23-, IL-12 and/or IFNα-associated conditions may comprise administering compounds of Formula I alone or in combination with each other and/or other suitable therapeutic agents useful in treating such conditions. Accordingly, "therapeutically effective amount" is also intended to include an amount of the combination of compounds claimed that is effective to inhibit IL-23, IL-12 and/or IFNα function and/or treat diseases associated with IL-23, IL-12 and/or IFNα.

Exemplary of such other therapeutic agents include corticosteroids, rolipram, calphostin, cytokine-suppressive anti-inflammatory drugs (CSAIDs), Interleukin-10, glucocorticoids, salicylates, nitric oxide, and other immunosuppressants; nuclear translocation inhibitors, such as deoxyspergualin (DSG); non-steroidal anti-inflammatory drugs (NSAIDs) such as ibuprofen, celecoxib and rofecoxib; steroids such as prednisone or dexamethasone; antiviral agents such as abacavir; antiproliferative agents such as methotrexate, leflunomide, FK506 (tacrolimus, PROGRAF^{®}); anti-malarials such as hydroxychloroquine; cytotoxic drugs such as azathiprine and cyclophosphamide; TNF-α inhibitors such as tenidap, anti-TNF antibodies or soluble TNF receptor, and rapamycin (sirolimus or RAPAMUNE^{®}) or derivatives thereof.

The above other therapeutic agents, when employed in combination with the compounds of the present invention, may be used, for example, in those amounts indicated in the *Physicians' Desk Reference* (PDR) or as otherwise determined by one of ordinary skill in the art. In the methods of the present invention, such other therapeutic agent(s) may be administered prior to, simultaneously with, or following the administration of the inventive compounds. The present invention also provides pharmaceutical compositions capable of treating IL-23-, IL-12- or IFNα-associated conditions by inhibiting Tyk2-mediated signal transduction, including IL-23-, IL-12- and/or IFNα-mediated diseases, as described above.

The inventive compositions may contain other therapeutic agents as described above and may be formulated, for example, by employing conventional solid or liquid vehicles or diluents, as well as pharmaceutical additives of a type appropriate to the mode of desired administration (*e.g.*, excipients, binders, preservatives, stabilizers, flavors, etc.) according to techniques such as those well known in the art of pharmaceutical formulation.

Accordingly, the present invention further includes compositions comprising one or more compounds of Formula I and a pharmaceutically acceptable carrier.

A "pharmaceutically acceptable carrier" refers to media generally accepted in the art for the delivery of biologically active agents to animals, in particular, mammals. Pharmaceutically acceptable carriers are formulated according to a number of factors well within the purview of those of ordinary skill in the art. These include without limitation the type and nature of the active agent being formulated; the subject to which the agent-containing composition is to be administered; the intended route of administration of the composition; and, the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, *e.g.,* stabilization of the active agent, binders, etc., well known to those of ordinary skill in the art. Descriptions of suitable pharmaceutically acceptable carriers, and factors involved in their selection, are found in a variety of readily available sources such as, for example, Remington's Pharmaceutical Sciences, 17th Edition (1985).

The compounds of Formula I may be administered by any means suitable for the condition to be treated, which may depend on the need for site-specific treatment or quantity of drug to be delivered. Topical administration is generally preferred for skin-related diseases, and systemic treatment preferred for cancerous or pre-cancerous conditions, although other modes of delivery are contemplated. For example, the compounds may be delivered orally, such as in the form of tablets, capsules, granules, powders, or liquid formulations including syrups; topically, such as in the form of solutions, suspensions, gels or ointments; sublingually; bucally; parenterally, such as by subcutaneous, intravenous, intramuscular or intrasternal injection or infusion techniques (*e.g.,* as sterile injectable aq. or non-aq. solutions or suspensions); nasally such as by inhalation spray; topically, such as in the form of a cream or ointment; rectally such as in the form of suppositories; or liposomally. Dosage unit formulations containing non-toxic, pharmaceutically acceptable vehicles or diluents may be administered. The compounds may be administered in a form suitable for immediate release or extended release. Immediate release or extended release may be achieved with suitable pharmaceutical compositions or, particularly in the case of extended release, with devices such as subcutaneous implants or osmotic pumps.

Exemplary compositions for topical administration include a topical carrier such as PLASTIBASE^{®} (mineral oil gelled with polyethylene).

Exemplary compositions for oral administration include suspensions which may contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which may contain, for example, microcrystalline cellulose, dicalcium phosphate, starch, magnesium stearate and/or lactose and/or other excipients, binders, extenders, disintegrants, diluents and lubricants such as those known in the art. The inventive compounds may also be orally delivered by sublingual and/or buccal administration, *e.g.,* with molded, compressed, or freeze-dried tablets. Exemplary compositions may include fast-dissolving diluents such as mannitol, lactose, sucrose, and/or cyclodextrins. Also included in such formulations may be high molecular weight excipients such as celluloses (AVICEL^{®}) or polyethylene glycols (PEG); an excipient to aid mucosal adhesion such as hydroxypropyl cellulose (HPC), hydroxypropyl methyl cellulose (HPMC), sodium carboxymethyl cellulose (SCMC), and/or maleic anhydride copolymer (*e.g.*, GANTREZ^{®}); and agents to control release such as polyacrylic copolymer (*e.g.*, CARBOPOL 934^{®}). Lubricants, glidants, flavors, coloring agents and stabilizers may also be added for ease of fabrication and use.

Exemplary compositions for nasal aerosol or inhalation administration include solutions which may contain, for example, benzyl alcohol or other suitable preservatives, absorption promoters to enhance absorption and/or bioavailability, and/or other solubilizing or dispersing agents such as those known in the art.

Exemplary compositions for parenteral administration include injectable solutions or suspensions which may contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid.

Exemplary compositions for rectal administration include suppositories which may contain, for example, suitable non-irritating excipients, such as cocoa butter, synthetic glyceride esters or polyethylene glycols, which are solid at ordinary temperatures but liquefy and/or dissolve in the rectal cavity to release the drug.

The therapeutically-effective amount of a compound of the present invention may be determined by one of ordinary skill in the art, and includes exemplary dosage amounts for a mammal of from about 0.05 to 1000 mg/kg; 1-1000 mg/kg; 1-50 mg/kg; 5-250 mg/kg; 250-1000 mg/kg of body weight of active compound per day, which may be administered in a single dose or in the form of individual divided doses, such as from 1 to 4 times per day. It will be understood that the specific dose level and frequency of dosage for any particular subject may be varied and will depend upon a variety of factors, including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats, horses, and the like. Thus, when the term "patient" is used herein, this term is intended to include all subjects, most preferably mammalian species that are affected by modulation of IL-23, IL-12 and/or IFNα-mediated functions.

### METHODS OF PREPARATION

The compounds of the present invention may be synthesized by many methods available to those skilled in the art of organic chemistry. General synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds. Examples of compounds of the present invention prepared by methods described in the general schemes are given in the preparations and examples section set out hereinafter.

### EXAMPLES

Preparation of compounds of Formula (I), and intermediates used in the preparation of compounds of Formula (I), can be prepared using procedures shown in the following Examples and related procedures. The methods and conditions used in these examples, and the actual compounds prepared in these Examples, are not meant to be limiting, but are meant to demonstrate how the compounds of Formula (I) can be prepared. Starting materials and reagents used in these examples, when not prepared by a procedure described herein, are generally either commercially available, or are reported in the chemical literature, or may be prepared by using procedures described in the chemical literature.

In the Examples given, the phrase "dried and concentrated" generally refers to drying of a solution in an organic solvent over either sodium sulfate or magnesium sulfate, followed by filtration and removal of the solvent from the filtrate (generally under reduced pressure and at a temperature suitable to the stability of the material being prepared). Column chromatography was performed with pre-packed silica gel cartridges using an Isco medium pressure chromatography apparatus (Teledyne Corporation), eluting with the solvent or solvent mixture indicated. The following abbreviations are used:

**Abbreviations**

| Abbreviation | |
|---|---|
| Ac | acetyl |
| ACN | acetonitrile |
| AcOH | acetic acid |
| anhyd. | anhydrous |
| aq. | aqueous |
| Bn | benzyl |
| Bu | butyl |
| Boc | tert-butoxycarbonyl |
| BOP | benzotriazol-1-yloxytris-(dimethylamino)-phosphonium hexafluorophosphate |
| CV | Column Volumes |
| DCE | dichloroethane |
| DCM | dichloromethane |
| DIC | *N,N,N'N'*-diisopropylcarbodiimide |
| DIPEA | diisopropylethylamine |
| DMB | 2,4-dimethoxybenzyl |
| DMF | dimethylformamide |
| DMSO | dimethylsulfoxide |
| EtOAc | ethyl acetate |
| Et | ethyl |
| EtOH | ethanol |
| H or H₂ | hydrogen |
| h, hr or hrs | hour(s) |
| HATU | O-(7-azabenzotriazol-1-yl)-N, N, N', N'-tetramethyluronium hexafluorophosphate |
| hex | hexane |
| i | iso |
| IPA | isopropyl alcohol |
| ISCO | automated chromatography |
| HOAc | acetic acid |
| HCl | hydrochloric acid |
| HPLC | high pressure liquid chromatography |
| LC | liquid chromatography |
| LIHMDS | Lithium bis(trimethylsilyl)amide |
| M | molar |
| mM | millimolar |
| Me | methyl |
| MeOH | methanol |
| MHz | megahertz |
| min. | minute(s) |
| mins | minute(s) |
| M+1 | (M+H)+ |
| MS | mass spectrometry |
| n or N | normal |
| nm | nanometer |
| nM | nanomolar |
| NMP | N-methylpyrrolidine |
| Pd/C | palladium on carbon |
| PdCl₂(dppf)₂ | [1,1'-bis(diphenylphosphino)f errocene]dichloropalladium(II) |
| Pd₂dba₃ | Tris(dibenzylideneacetone)dipalladium(0) |
| Ph | phenyl |
| PPh₃ | triphenylphosphine |
| Pr | propyl |
| PSI | pounds per square inch |
| rb | round bottom |
| rt | room temperature |
| Ret Time | retention time |
| sat. | saturated |
| SFC | supercritical fluid chromatography |
| TBAF | tetrabutylammonium fluoride |
| TEA | triethylamine |
| TFA | trifluoroacetic acid |
| THF | tetrahydrofuran |
| Xantphos | 4,5-bis(diphenylphosphino)-9,9-dimethylxanthene |

### Preparations

The preparations set out below are for the synthesis of reagents that were not obtained from commercial sources and were employed for the preparation of compounds of formula I of the invention. All chiral compounds in the Tables and Schemes are racemic unless specified otherwise.

Reverse-phase preparative high performance liquid chromatography ("HPLC") was performed with Shimadzu 8A liquid chromatographs using YMC S5 ODS columns (20 x 100, 20 x 250, or 30 x 250 millimeter ("mm")). Gradient elution was performed with methanol ("MeOH")/water mixtures in the presence of 0.1% trifluoroacetic acid ("TFA").

### HPLC Methods

### Method A: (analytical)

Column: Waters Acquity BEH C₁₈ 2.0 x 50 mm, 1.7 µm; mobile phase A: water with 0.1% TFA; mobile phase B: MeCN with 0.1% TFA; temperature: 40 °C; flow rate 1 mL/min; gradient: 0-100% B over 1.5 min, then 0.5 min isocratic at 100% B.

### Method B: (analytical)

Column: Acquity UPLC^{®} BEH C₁₈ 2.1 x 50 mm, 1.7 µm (Waters Corp.); mobile phase A: water with 0.05% TFA; mobile phase B: MeCN with 0.05% TFA; temperature: 50 °C; flow rate 0.8 mL/min; gradient: 0-100% B over 1.8 min.

### Method C: (analytical)

Column: Acquity UPLC^{®} BEH C₁₈ 2.1 x 50 mm, 1.7 µm (Waters Corp.); mobile phase A: water with 0.1% TFA; mobile phase B: MeCN with 0.1% TFA; temperature: 50 °C; flow rate 1 mL/min; gradient: 0-100% B over 3 min, then 0.5 min isocratic at 100% B.
Method D: (QC-ACN-AA-XB) Waters Acquity UPLC BEH C18, 2.1 x 50 mm, 1.7-µm particles; Mobile Phase A: 5:95 acetonitrile:water with 10 mM ammonium acetate; Mobile Phase B: 95:5 acetonitrile:water with 10 mM ammonium acetate; Temperature: 50 °C; Gradient: 0-100% B over 3 minutes, then a 0.75-minute hold at 100% B; Flow: 1.0 mL/min; Detection: UV at 220 nm.
Method E: (QC-ACN-TFA-XB) Column: Waters Acquity UPLC BEH C18, 2.1 x 50 mm, 1.7 µm particles; Mobile Phase A: 5:95 acetonitrile:water with 0.1% trifluoroacetic acid; Mobile Phase B: 95:5 acetonitrile:water with 0.1% trifluoroacetic acid; Temperature: 50 °C; Gradient: 0-100% B over 3 minutes, then a 0.75-minute hold at 100% B; Flow: 1.0 mL/min; Detection: UV at 220 nm.
Method F: Column: Waters Acquity UPLC BEH C18 (2.1 x 50 mm), 1.7 micron; Solvent A = 100% water with 0.05% TFA; Solvent B = 100% acetonitrile with 0.05% TFA; gradient = 2-98% B over 1 minute, then a 0.5 minute hold at 98% B; Flow rate: 0.8 mL/min;

### Example 1

### Methyl (6-carbamoyl-5-((2-methoxy-3-(1-methyl-1H-1,2,4-triazol-3-yl)phenyl)amino)pyridazin-3-yl)carbamate

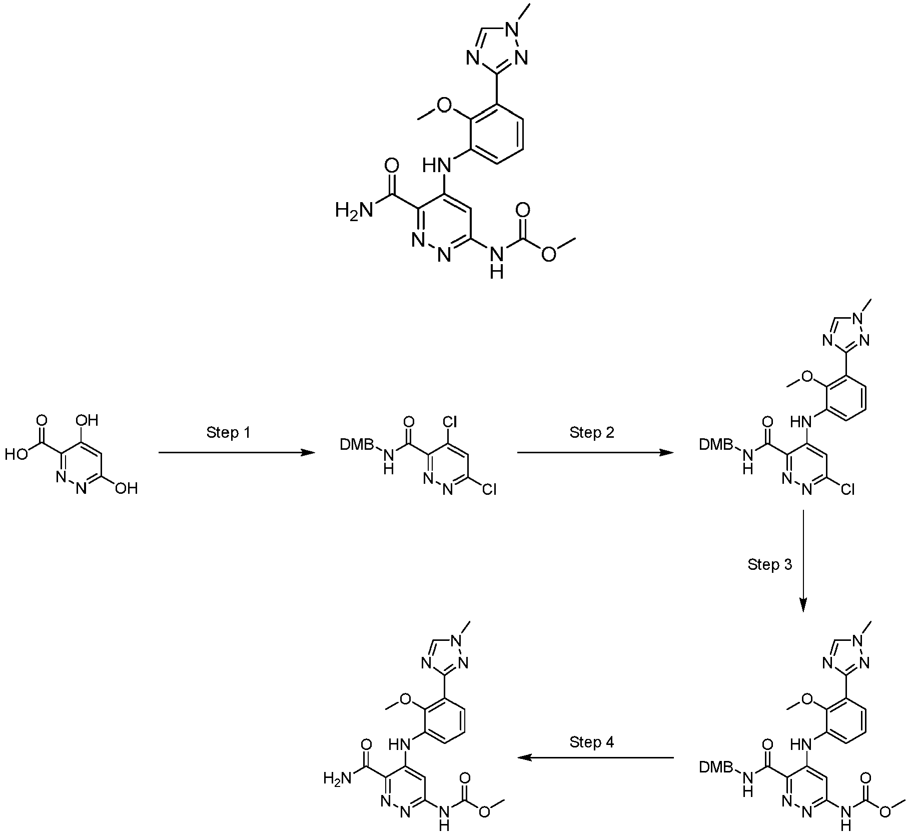

### Step 1:

POCl₃ (11.9 mL, 128 mmol) was added to 4,6-dihydroxypyridazine-3-carboxylic acid (4.0 g, 25.6 mmol) in a 250 mL round bottom flask. Triethylamine (3.57 mL, 25.6 mmol) was added dropwise. The vessel was fitted with a reflux condenser and the reaction was heated to 50 °C. After several minutes, refluxing ensued. After refluxing ended, the reaction was heated to 90 °C for 2 hours. The reaction was then cooled to room temperature and concentrated. Toluene (18 mL) was added to make a slurry which was concentrated under vacuum; this process was repeated twice. Next, DCM (100 mL) was added followed by triethylamine (14.3 mL, 103 mmol), which caused the reaction to reflux quickly. The reaction mixture was cooled to 0 °C and (2,4-dimethoxyphenyl)methanamine (11.6 mL, 77 mmol) was added slowly, causing the reaction to bubble as internal warming caused solvent to reflux. The reaction was warmed to room temperature and stirred for 30 minutes. The reaction mixture was diluted with DCM and washed with saturated aqueous potassium phosphate dibasic solution and water. The organic layer was dried over magnesium sulfate, filtered and concentrated. The crude material was dissolved in DCM and concentrated onto celite, which was loaded into an empty plastic cartridge for purification on silica gel using Hexanes/EtOAc 0-100%. The fractions containing the desired product were concentrated to afford 4,6-dichloro-N-(2,4-dimethoxybenzyl)pyridazine-3-carboxamide (2.5 g, 7.31 mmol, 28.5 % yield). LCMS *m*/*z* 341.9 (M+H)⁺; HPLC *t*_{R} 0.85 min (Method F).

### Step 2:

To a solution of 4,6-dichloro-*N*-(2,4-dimethoxybenzyl)pyridazine-3-carboxamide (75 mg, 0.219 mmol) and 2-methoxy-3-(1-methyl-1*H*-1,2,4-triazol-3-yl)aniline (49.2 mg, 0.241 mmol) in THF (1.1 mL) was added LiHMDS in THF (1M, 70 µl, 0.70 mmol) at room temperature. The reaction was quenched after 15 minutes by addition of saturated aqueous ammonium chloride solution, water, and DCM. The reaction was extracted with DCM, and the combined organic layer was dried over sodium sulfate, filtered, and concentrated to give 6-chloro-*N*-(2,4-dimethoxybenzyl)-4-((2-methoxy-3-(1-methyl-1*H-*1,2,4-triazol-3-yl)phenyl)amino)pyridazine-3-carboxamide. The material was considered quantitative (0.219 mmol) and used as such. LCMS *m*/*z* 510.1 (M+H)⁺; HPLC *t*_{R} 0.93 min (Method F).

### Step 3:

A mixture of 6-chloro-*N*-(2,4-dimethoxybenzyl)-4-((2-methoxy-3-(1-methyl-1*H-*1,2,4-triazol-3-yl)phenyl)amino)pyridazine-3-carboxamide (0.109 mmol, ½ of the material from Step 2), methyl carbamate (0.041 g, 0.545 mmol), chloro(2-dicyclohexylphosphino-2'4'6'-triisopropyl-1,1'-bipheyl)[2-(2'-amino-1,1'-biphenyl)]palladium(II) (0.017 g, 0.022 mmol) and Cs₂CO₃ (0.089 g, 0.273 mmol) in 1,4-dioxane (1 mL) was degassed by bubbling nitrogen gas through the mixture for 10 minutes. The reaction vessel was sealed and heated to 100 °C for 2 hours. Upon completion, the reaction was quenched by diluting with water and DCM. The aqueous layer was extracted twice with DCM. The combined organic layer was dried over sodium sulfate, filtered, and concentrated to afford a crude solid which was considered quantitative recovery of methyl (6-((2,4-dimethoxybenzyl)carbamoyl)-5-((2-methoxy-3-(1-methyl-1*H*-1,2,4-triazol-3-yl)phenyl)amino)pyridazin-3-yl)carbamate (0.109 mmol) and used as such. LCMS *m*/*z* 549.2 (M+H)⁺; HPLC *t*_{R} 0.80 min (Method F).

### Step 4: Example 1

A solution of the material from Step 3 methyl (6-((2,4-dimethoxybenzyl)carbamoyl)-5-((2-methoxy-3-(1-methyl-1*H*-1,2,4-triazol-3-yl)phenyl)amino)pyridazin-3-yl)carbamate (0.109 mmol) in TFA (1.5 mL) was heated to 60 °C for 30 minutes. Upon completion, the reaction was concentrated, taken up in DCM, and neutralized by washing with saturated aqueous NaHCO₃. The organic layer was dried over sodium sulfate, filtered, concentrated, and taken up in DMF for purification. The crude material was purified via preparative LC/MS with the following conditions: Column: XBridge C18, 19 x 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 15-45% B over 20 minutes, then a 5-minute hold at 100% B; Flow: 20 mL/min. Fractions containing the desired product were combined and dried via centrifugal evaporation to give methyl (6-carbamoyl-5-((2-methoxy-3-(1-methyl-1H-1,2,4-triazol-3-yl)phenyl)amino)pyridazin-3-yl)carbamate (8.1 mg, 0.020 mmol, 18.28 % yield). LCMS *m*/*z* 399.3 (M+H)⁺; HPLC *t*_{R} 0.75 min (QC-ACN-TFA-XB). ¹H NMR (500MHz, DMSO-d₆) δ 11.03 (s, 1H), 10.83 (s, 1H), 8.57 (s, 1H), 8.51 (br. s., 1H), 7.95 - 7.78 (m, 2H), 7.67 (d, *J*=7.7 Hz, 1H), 7.54 (d, *J*=7.7 Hz, 1H), 7.29 (t, *J*=7.8 Hz, 1H), 3.95 (s, 3H), 3.71 (s, 3H), 3.67 (s, 3H).

### Reference Example 2

### 6-(3-ethylureido)-4-((2-methoxy-3-(5-methyl-1,2,4-oxadiazol-3-yl)phenyl)amino)-N-(trideuteromethyl)pyridazine-3-carboxamide

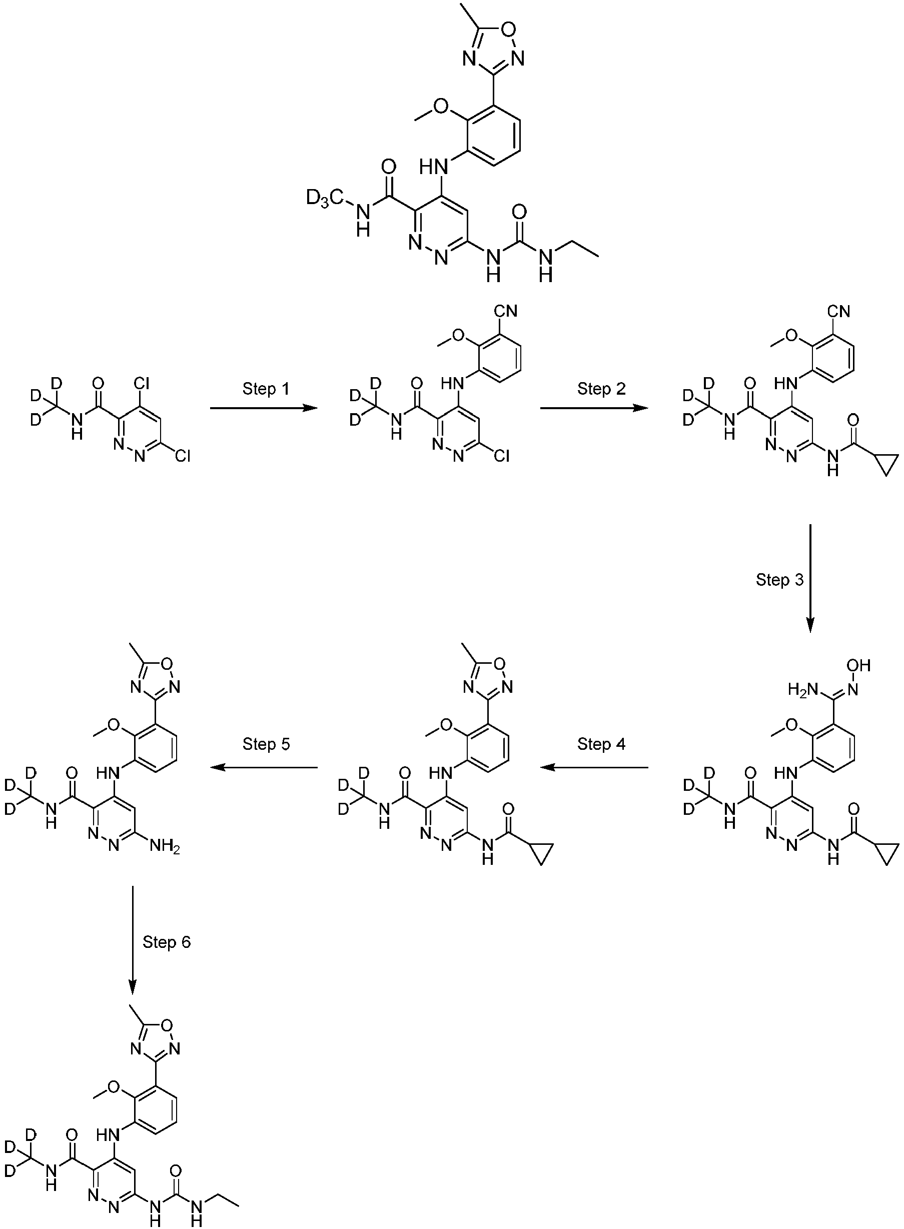

### Step 1:

To a solution of 4,6-dichloro-*N*-trideuteromethylpyridazine-3-carboxamide (605 mg, 2.89 mmol) and 3-amino-2-methoxybenzonitrile (472 mg, 3.18 mmol) in THF (15 mL) was added LiHMDS (0.5M in 2-MeTHF, 18.5 mL, 9.25 mmol). The reaction vial was stirred at 25 °C for 35 minutes. Upon completion, the reaction was quenched via addition of saturated aqueous ammonium chloride solution, water, and DCM. The aqueous layer was extracted with DCM. The combined organic layer was dried over sodium sulfate, filtered, and concentrated to give crude 6-chloro-4-((3-cyano-2-methoxyphenyl)amino)-*N*-trideuteromethylpyridazine-3-carboxamide. Considered quantitative (2.89 mmol) and carried forward as such. LCMS *m*/*z* 321.0 (M+H)⁺; HPLC *t*_{R} 0.84 min (Method F).

### Step 2:

The material from Step 1 (6-chloro-4-((3-cyano-2-methoxyphenyl)amino)-*N-*trideuteromethylpyridazine-3-carboxamide (2.89 mmol)), cyclopropanecarboxamide (1.23 g, 14.5 mmol), Pd₂(dba)₃ (0.265 g, 0.289 mmol), Xantphos (0.334 g, 0.578 mmol) and Cs₂CO₃ (2.354 g, 7.23 mmol) in 1,4-dioxane (15 mL) was degassed by bubbling nitrogen gas through the mixture for 5 minutes. The reaction vessel was sealed and heated to 130 °C for 45 minutes. Upon completion, the reaction mixture was diluted with DCM, filtered through a celite pad, and concentrated. The crude isolate was then purified by column chromatography on silica gel loading in DMF and eluting with DCM/MeOH 0-10% to give fractions containing water-soluble impurities. The desired fractions were combined and washed with water five times, dried over sodium sulfate, filtered, and concentrated to afford 4-((3-cyano-2-methoxyphenyl)amino)-6-(cyclopropanecarboxamido)-*N*-trideuteromethylpyridazine-3-carboxamide. Material was assumed quantitative (2.89 mmol) and used as such. LCMS *m*/*z* 370.1 (M+H)⁺; HPLC *t*_{R} 0.78 min (Method F). ¹H NMR (400 MHz, DMSO-d₆) δ 11.37 (s, 1H), 10.99 (s, 1H), 9.17 (s, 1H), 8.05 (s, 1H), 7.78 (dd, *J*=8.0, 1.4 Hz, 1H), 7.61 (dd, *J*=7.9, 1.5 Hz, 1H), 7.36 (t, *J*=7.9 Hz, 1H), 3.91 (s, 3H), 2.13 - 2.01 (m, 1H), 0.90 - 0.75 (m, 4H)

### Step 3:

To a mixture of 4-((3-cyano-2-methoxyphenyl)amino)-6-(cyclopropanecarboxamido)*-N*-trideuteromethylpyridazine-3-carboxamide (1.04 mmol) and hydroxylamine hydrochloride (367 mg, 5.28 mmol) in EtOH (15 mL) was added potassium hydroxide (285 mg, 5.07 mmol). The mixture was sealed and heated to 85 °C. After 19 hours, another aliquot of hydroxylamine hydrochloride (367 mg, 5.28 mmol) and potassium hydroxide (285 mg, 5.07 mmol) were each added and the reaction was heated for 8 hours more. Upon completion, the reaction was cooled to room temperature. The reaction was diluted with DCM and poured into water. The aqueous layer was extracted with DCM three times, and then the combined organic layer was dried over sodium sulfate, filtered, and concentrated to afford a crude brown solid. This material was dissolved in a mixture of DCM and MeOH, loaded onto silica gel, and purified by column chromatography eluting with DCM/MeOH 0-15%. Concentration of fractions containing the desired product provided (*Z*)-6-(cyclopropanecarboxamido)-4-((3-(*N'-*hydroxycarbamimidoyl)-2-methoxyphenyl)amino)-*N*-trideuteromethylpyridazine-3-carboxamide (260 mg, 0.646 mmol, 62.4 % yield). LCMS *m*/*z* 403.1 (M+H)⁺; HPLC *t*_{R} 0.54 min (analytical HPLC Method TS1).

### Step 4:

To a solution of (Z)-6-(cyclopropanecarboxamido)-4-((3-(N'-hydroxycarbamimidoyl)-2-methoxyphenyl)amino)-N-trideuteromethylpyridazine-3-carboxamide (147 mg, 0.365 mmol) and acetic acid (0.031 mL, 0.548 mmol) in DMF (3 mL) at room temperature was added DIC (0.114 mL, 0.731 mmol). After 90 minutes, another aliquot each of acetic acid (0.031 mL, 0.548 mmol) and DIC (0.114 mL, 0.731 mmol) were added and the reaction was stirred for another hour. Then, TBAF (1M in THF, 1.83 mL, 1.83 mmol) was added in a single portion. After 55 minutes, another aliquot of TBAF (1M in THF, 1.83 mL, 1.83 mmol) was added, and the reaction was finished 20 minutes later. Upon completion, the reaction was diluted with DCM, water, and 1.5 mL of saturated aqueous NaHCO₃ solution. The organic layer was washed with water three times and then dried over sodium sulfate, filtered, and concentrated to afford crude brown material. This material was dissolved in DMF and loaded onto silica gel for purification by column chromatography eluting with DCM/MeOH 0-15%. Concentration of fractions containing the desired product provided 6-(cyclopropanecarboxamido)-4-((2-methoxy-3-(5-methyl-1,2,4-oxadiazol-3-yl)phenyl)amino)-N-trideuteromethylpyridazine-3-carboxamide (116 mg, 0.272 mmol, 74.5 % yield). LCMS m/z 427.1 (M+H)⁺; HPLC *t*_{R} 0.76 min (Method F).

### Step 5:

To 6-(cyclopropanecarboxamido)-4-((2-methoxy-3-(5-methyl-1,2,4-oxadiazol-3-yl)phenyl)amino)-*N*-trideuteromethylpyridazine-3-carboxamide (39 mg, 0.091 mmol) in EtOH (0.92 mL) was added potassium hydroxide (15.4 mg, 0.274 mmol) and the reaction mixture was sealed and heated to 85 °C for 80 minutes. Upon completion, the reaction was quenched with saturated aqueous ammonium chloride solution and water. The crude mixture was extracted three times with CHCl₃/iPrOH 4/1, and the combined organic extracts were dried over sodium sulfate, filtered, and concentrated to obtain 6-amino-4-((2-methoxy-3-(5-methyl-1,2,4-oxadiazol-3-yl)phenyl)amino)-N-(trideuteromethyl)pyridazine-3-carboxamide. Material was assumed quantitative (0.091 mmol) and used as such. LCMS *m*/*z* 359.0 (M+H)⁺; HPLC *t*_{R} 0.63 min (Method F).

### Step 6:

The material from Step 5 (6-amino-4-((2-methoxy-3-(5-methyl-1,2,4-oxadiazol-3-yl)phenyl)amino)-*N*-trideuteromethylpyridazine-3-carboxamide (0.091 mmol)) was dissolved in DMSO (0.91 mL) and TEA (63.4 µl, 0.455 mmol) and isocyanatoethane (14.1 µl, 0.182 mmol) were added. The reaction was heated to 100 °C for 2 hours and 15 minutes, and then another aliquot of isocyanatoethane (28.1 µl, 0.364 mmol) was added. After another hour stirring at 100 °C, the reaction was cooled to room temperature, diluted with a few drops of water to quench excess reagent, and 1.1 mL of DMSO was added. The crude material was purified via preparative LC/MS with the following conditions: Column: XBridge C18, 19 x 200 mm, 5-µm particles; Mobile Phase A: 5:95 acetonitrile: water with 0.1% trifluoroacetic acid; Mobile Phase B: 95:5 acetonitrile: water with 0.1% trifluoroacetic acid; Gradient: 10-50% B over 20 minutes, then a 5-minute hold at 100% B; Flow: 20 mL/min. The fractions containing the desired product were combined and dried via centrifugal evaporation to afford 6-(3-ethylureido)-4-((2-methoxy-3-(5-methyl-1,2,4-oxadiazol-3-yl)phenyl)amino)-*N*-trideuteromethylpyridazine-3-carboxamide, TFA (18.7 mg, 0.032 mmol, 35.2 % yield). LCMS *m*/*z* 430.1 (M+H)⁺; HPLC *t*_{R} 1.11 min (QC-ACN-TFA-XB). ¹H NMR (500MHz, DMSO-d₆) δ 10.97 (s, 1H), 9.46 (s, 1H), 9.10 (s, 1H), 7.74 - 7.60 (m, 3H), 7.44 (br. s., 1H), 7.37 (t, *J*=7.9 Hz, 1H), 3.71 (s, 3H), 3.19 - 3.06 (m, 2H), 2.67 (s, 3H), 1.04 (t, *J*=7.2 Hz, 3H).

### Reference Example 3

### 4-((2-methoxy-3-(5-methyl-1,2,4-oxadiazol-3-yl)phenyl)amino)-N-(trideuteromethyl)-6-(3-methylureido)pyridazine-3-carboxamide

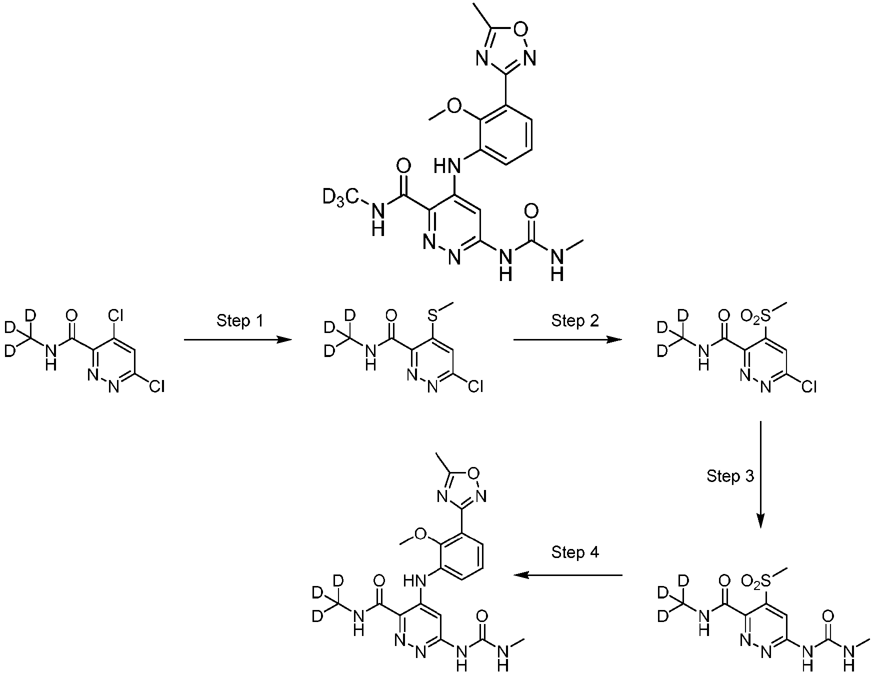

### Step 1:

To a solution of 4,6-dichloro-*N*-trideuteromethylpyridazine-3-carboxamide (0.200 g, 0.957 mmol) in THF (8 mL) at 0 °C was added a chilled solution (0 °C) of sodium methanethiolate (0.067 g, 0.957 mmol) in water (1.6 mL) dropwise. After 5 minutes, another aliquot of sodium methanethiolate (0.017 g, 0.239 mmol) in water (0.4 mL) was added. After 5 minutes more, another aliquot of sodium methanethiolate (0.017 g, 0.239 mmol) in water (0.4 mL) was added. The reaction was stirred for 5 minutes and then quenched via addition of water and DCM. The aqueous layer was extracted with DCM three times, and the combined organic layer was dried over sodium sulfate, filtered, and concentrated to afford 6-chloro-*N*-trideuteromethyl-4-(methylthio)pyridazine-3-carboxamide (205 mg, 0.929 mmol, 97 % yield). Carried forward as is. LCMS *m*/*z* 220.9 (M+H)⁺; HPLC *t*_{R} 0.63 min (Method F).

### Step 2:

6-chloro-*N*-trideuteromethyl-4-(methylthio)pyridazine-3-carboxamide (0.205 g, 0.929 mmol) was dissolved in DCM (9.3 mL) and *m*CPBA (77%, 0.833 g, 3.72 mmol) was added in a single portion. The reaction was stirred for 90 minutes. Upon completion, solids were filtered off and remaining crude material was loaded onto silica gel in DCM with trace methanol for column chromatography eluting with Hex/EtOAc 0-100% to give 6-chloro-*N*-trideuteromethyl-4-(methylsulfonyl)pyridazine-3-carboxamide (198 mg, 0.784 mmol, 84 % yield). LCMS *m*/*z* 252.9 (M+H)⁺; HPLC *t*_{R} 0.53 min (Method F).

### Step 3:

A mixture of 6-chloro-*N*-trideuteromethyl-4-(methylsulfonyl)pyridazine-3-carboxamide (40 mg, 0.158 mmol), Xantphos (18.3 mg, 0.032 mmol), Pd₂(dba)₃ (14.5 mg, 0.016 mmol), 1-methylurea (17.6 mg, 0.237 mmol) and Cs₂CO₃ (129 mg, 0.396 mmol) in 1,4-dioxane (1.6 mL) was degassed by bubbling nitrogen gas through the mixture for 10 minutes. The reaction vessel was sealed and heated to 70 °C for 30 minutes. Upon completion, the reaction was cooled to room temperature, filtered through a pad of celite, taken up in DCM with a trace of methanol, and loaded onto silica gel for purification by column chromatography eluting with DCM/MeOH 0-10% to give *N-*trideuteromethyl-4-(methylsulfonyl)-6-(3-methylureido)pyridazine-3-carboxamide (20 mg, 0.069 mmol, 43.5 % yield). LCMS *m*/*z* 291.0 (M+H)⁺; HPLC *t*_{R} 0.48 min (Method F).

### Step 4:

To a mixture of 2-methoxy-3-(5-methyl-1,2,4-oxadiazol-3-yl)aniline (14.1 mg, 0.069 mmol, prepared similarly to Steps 3 and 4 in Example C) and *N*-trideuteromethyl-4-(methylsulfonyl)-6-(3-methylureido)pyridazine-3-carboxamide (10 mg, 0.034 mmol) in EtOH (0.6 mL) at room temperature was added HCl (4M in 1,4-dioxane, 10 µl, 0.040 mmol). The reaction was heated to 100 °C for 17 hours. Upon completion, the reaction was concentrated, taken up in 2 mL of DMSO with a few drops of Et₃N to neutralize any residual HCl, and the solution was purified by preparative LC/MS with the following conditions: Column: XBridge C18, 19 x 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 15-100% B over 20 minutes, then a 5-minute hold at 100% B; Flow: 20 mL/min. The fractions containing the desired product were combined and dried via centrifugal evaporation to provide 4-((2-methoxy-3-(5-methyl-1,2,4-oxadiazol-3-yl)phenyl)amino)-*N*-(trideuteromethyl)-6-(3-methylureido)pyridazine-3-carboxamide (5.0 mg, 0.011 mmol, 32.1 % yield). LCMS *m*/*z* 415.9 (M+H)⁺; HPLC *t*_{R} 1.41 min (QC-ACN-AA-XB). ¹H NMR (500MHz, DMSO-d₆) δ 10.99 (s, 1H), 9.55 (s, 1H), 9.11 (s, 1H), 7.72 (s, 1H), 7.71 - 7.63 (m, 2H), 7.37 (t, *J*=7.9 Hz, 1H), 7.29 (br. s., 1H), 3.73 (s, 3H), 2.68 (s, 3H), 2.67 (s, 3H).

### Example 4

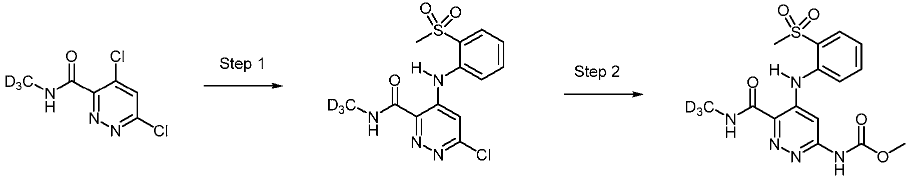

### Step 1:

To a clear solution of 2-(methylsulfonyl)aniline (164 mg, 0.957 mmol) and 4,6-dichloro-N-(methyl-d3)pyridazine-3-carboxamide (200 mg, 0.957 mmol) in THF (10 ml) was added potassium tert-butoxide (322 mg, 2.87 mmol) and stirred at 65 °C for 2h. The reaction was quenched with sat. NH₄Cl and the pH was ajusted to 7.0 with 1N HCl, and extracted with EtOAc (2x). The organic layer was collected, dried over Na₂SO₄, concentrated to give 6-chloro-N-(methyl-d3)-4-((2-(methylsulfonyl)phenyl)amino)pyridazine-3-carboxamide, M+H=344. *t*_{R} =1.62 min. LCMS method: Start % B = 0, Final % B = 100, Gradient Time = 4 min, Flow Rate = 4 ml/min, Wavelength = 220, Solvent Pair = ACN-H2O-NH4OAC, Solvent A = 5:95 ACN:water with 10-mM NH4OAc, Solvent B = 95:5 ACN:water with 10-mM NH4OAc, Column 1 = 1: Waters Xbridge C18 4.6 x 50 mm 5 um, Oven Temp. = 35. ¹H NMR (500 MHz, DMSO-d₆) δ 11.23 - 11.16 (m, 1H), 9.41 - 9.32 (m, 1H), 8.06 - 7.93 (m, 1H), 7.85 - 7.72 (m, 2H), 7.60 - 7.48 (m, 1H), 7.31 - 7.19 (m, 1H), 3.20 (s, 3H).

### Step 2:

To a solution of 6-chloro-N-(methyl-d3)-4-((2-(methylsulfonyl)phenyl) amino)pyridazine-3-carboxamide (20 mg, 0.058 mmol), methyl carbamate (13.10 mg, 0.175 mmol), Pd₂(dba)₃ (5.33 mg, 5.82 µmol), Xantphos (6.73 mg, 0.012 mmol), Cs₂CO₃ (37.9 mg, 0.116 mmol) in dioxane (1.0 mL) was sparged with nitrogen for 5 min., then was placed into a preheated 130 °C heating block for 1 h. The reaction was cooled down and diluted with DMF, filtered and concentrated to give methyl (6-((methyl-d3)carbamoyl)-5-((2-(methylsulfonyl)phenyl)amino)pyridazin-3-yl)carbamate, M+H=383, after purification by reverse phase HPLC. ¹H NMR (500 MHz, DMSO-d₆) δ 11.10 - 11.05 (m, 1H), 9.08 (s, 1H), 8.05 - 7.94 (m, 1H), 7.86 - 7.69 (m, 4H), 7.55 - 7.42 (m, 1H), 3.64 (s, 3H), 3.18 (s, 3H).

### Example 5

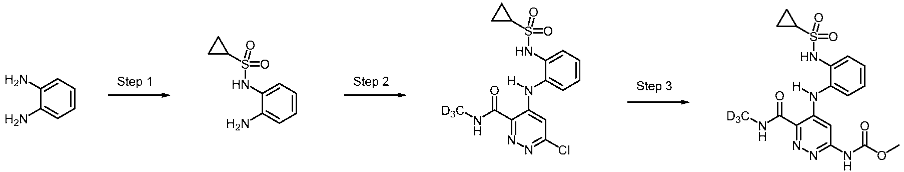

### Step 1:

To a solution of benzene-1,2-diamine (0.5 g, 4.62 mmol) in CH₂Cl₂ (30 mL) at 0 °C was added TEA (1.289 mL, 9.25 mmol) followed by dropwise addition of cyclopropanesulfonyl chloride (0.683 g, 4.85 mmol). The mixture was allowed to warm to room temperature and stirred for 2h. 1M HCl was added and the layers were separated. The aqueous layer was adjusted to pH=11 with solid NaOH, extracted with CH₂Cl₂. The basified aqueous layer was then neutralized using 3N HCl and extracted with CH₂Cl₂, dried with Na₂SO₄, filtered, and concentrated in vacuo to give N-(2-aminophenyl)cyclopropanesulfonamide, M+H=213. *t*_{R} = 0.75 min LCMS method: Start %B = 0, Final %B = 100, Gradient Time 1.5 min, Flow Rate 1 min/mL, Wavelength 220, Solvent Pair = Water: ACN: TFA, Solvent A = 90:10 Water: ACN with 0.1% TFA, Solvent B = 10:90 Water: ACN with 0.1% TFA, Column = 1: Waters Acquity BEH C18 1.7 um 2.0 x 50 mm.

### Step 2:

To a clear solution of 4,6-dichloro-N-(methyl-d3)pyridazine-3-carboxamide (423 mg, 2.026 mmol) and N-(2-aminophenyl)cyclopropanesulfonamide (430 mg, 2.026 mmol) in tetrahydrofuran (6 ml) was added lithium bis(trimethylsilyl)amide (6077 µl, 6.08 mmol) dropwise at 0°C to cause color changing to dark amber, the mixture was stirred at room temperature overnight. The mixture was quenched with water (1 mL) at 0°C and acidified with 1N HCl to pH = 5-6 and extracted with AcOEt (50 mL), the mixture was then extracted with 1N NaOH solution (50 mL), the aqueous layer was washed with AcOEt (20 mL), and acidified with conc. HCl to pH =5-6, extracted with AcOEt (50 mL), washed with brine, dried (Na₂SO₄) and concentrated to get the desired 6-chloro-4-((2-(cyclopropanesulfonamido)phenyl)amino)-N-(methyl-d3)pyridazine-3-carboxamide which was used as is, M+H=385. ¹H NMR (400 MHz, METHANOL-d₄) δ 7.67 - 7.54 (m, 1H), 7.51 - 7.31 (m, 3H), 6.98 - 6.86 (m, 1H), 2.79 - 2.65 (m, 1H), 1.04 - 0.90 (m, 4H)

### Step 3:

A mixture of 6-chloro-4-((2-(cyclopropanesulfonamido)phenyl)amino)-N-(methyl-d3)pyridazine-3-carboxamide (25 mg, 0.065 mmol), methyl carbamate (14.63 mg, 0.195 mmol), Pd₂(dba)₃ (5.95 mg, 6.50 µmol), Xantphos (7.52 mg, 0.013 mmol), Cs₂CO₃ (42.3 mg, 0.130 mmol) in dioxane (1.0 mL) was sparged with nitrogen for 5 min., then was placed into a preheated 130 °C heating block for 1 h. The reaction was cooled down and diluted with DMF, filtered and purified by reverse phase preparative HPLC to give methyl (5-((2-(cyclopropanesulfonamido)phenyl)amino)-6-((methyl-d3)carbamoyl)pyridazin-3-yl)carbamate, M+H=424. ¹H NMR (500 MHz, DMSO-d₆) δ 10.67 - 10.55 (m, 1H), 9.12 - 8.93 (m, 1H), 7.67 - 7.18 (m, 5H), 3.45 (s, 3H), 2.71 - 2.61 (m, 1H), 0.99 - 0.75 (m, 4H).

### Reference Example 6

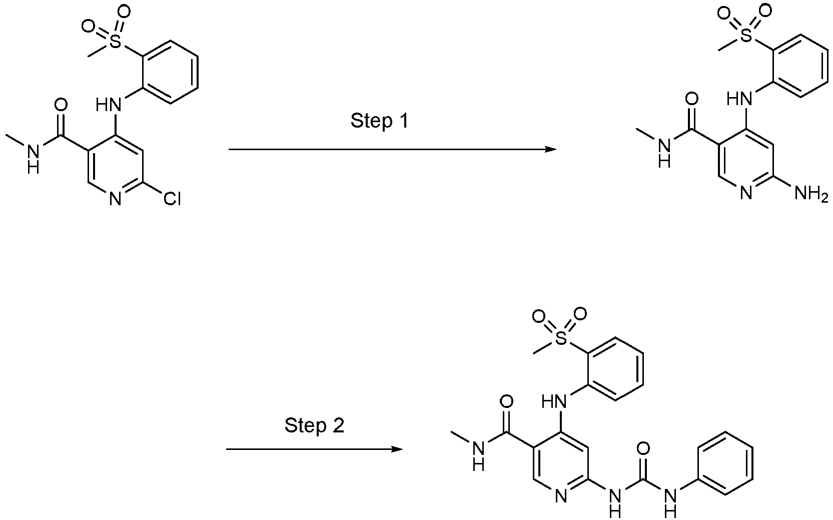

### Step 1:

6-chloro-N-methyl-4-((2-(methylsulfonyl)phenyl)amino)nicotinamide (30 mg, 0.088 mmol), 1,1-dimethylurea (11.67 mg, 0.132 mmol) were added to DMA (1 mL). It was stirred while purging with N₂ for 5 min. Pd₂(dba)₃ (8.08 mg, 8.83 µmol), Xantphos (10.22 mg, 0.018 mmol) and Cs₂CO₃ (57.5 mg, 0.177 mmol) were added to the reaction while still purging with N₂. The reaction was then sealed and heated at 145 °C for 2 h. The reaction did not give desired urea displaced product but gave 6-amino-N-methyl-4-((2-(methylsulfonyl)phenyl)amino)nicotinamide. LCMS retention time 0.54 min. M+1 = 321. Waters Acquity SDS: Column: BEH C18, 2.1 x 50 mm, 1.7 µm particles; Mobile Phase: (A) water; (B) acetonitrile; Buffer: 0.05% TFA; Gradient Range: 2%-98% B (0 to 1 min) 98%B (to 1.5 min) 98%-2% B (to 1.6 min); Gradient Time: 1.6 min; Flow Rate: 0.8 mL/min; Analysis Time: 1.7 min; Detection: Detector 1: UV at 220 nm; Detector 2: MS (ES+).

### Step 2:

6-amino-N-methyl-4-((2-(methylsulfonyl)phenyl)amino)nicotinamide (40 mg, 0.125 mmol) andisocyanatobenzene (14.87 mg, 0.125 mmol) were added to dioxane (1 mL). The reaction mixture was stirred overnight. The reaction was filtered and purified with reverse phase preparative HPLC. It gave the desired product N-methyl-4-((2-(methylsulfonyl)phenyl)amino)-6-(3-phenylureido)nicotinamide (15.8 mg, 0.033 mmol, 26.8% yield).
¹H NMR (500 MHz, METHANOL-d₄) δ 8.47 - 8.43 (m, 1H), 8.05 - 8.01 (m, 1H), 7.73 - 7.68 (m, 2H), 7.64 - 7.59 (m, 4H), 7.49 - 7.44 (m, 2H), 7.43 - 7.39 (m, 1H), 7.42 - 7.39 (m, 1H), 7.36 - 7.34 (m, 1H), 7.32 - 7.27 (m, 3H), 7.21 - 7.16 (m, 1H), 7.10 - 7.04 (m, 1H), 3.17 - 3.08 (m, 3H), 2.97 - 2.91 (m, 3H)

### Reference Example 7

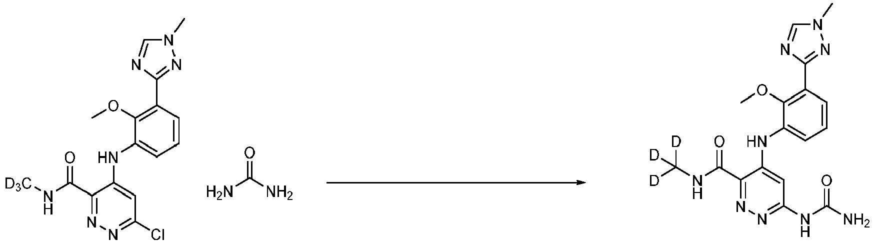

### Step 1:

6-chloro-4-((2-methoxy-3-(1-methyl-1H-1,2,4-triazol-3-yl)phenyl)amino)-N-(methyl-d3)pyridazine-3-carboxamide (25 mg, 0.066 mmol), urea (7.97 mg, 0.133 mmol), Pd₂(dba)₃ (6.08 mg, 6.63 µmol), Xantphos (5.76 mg, 9.95 µmol) and Cs₂CO₃ (43.2 mg, 0.133 mmol) were dissolved in dioxane (2.0 mL). It was degassed and backfilled with N₂. It was sealed and stirred at 130°C for 1.0 h. The reaction gave desired product. It was cooled to room temperature., filtered and purified with reverse phase preparative HPLC to provide 4-((2-methoxy-3-(1-methyl-1H-1,2,4-triazol-3-yl)phenyl)amino)-N-(methyl-d3)-6-ureidopyridazine-3-carboxamide (5.3 mg, 0.013 mmol, 19.55 % yield).
¹H NMR (500 MHz, DMSO-d₆) δ 11.07 - 10.93 (m, 1H), 9.62 - 9.44 (m, 1H), 9.21 - 9.06 (m, 1H), 8.58 (s, 1H), 7.84 - 7.72 (m, 1H), 7.69 - 7.62 (m, 1H), 7.57 - 7.50 (m, 1H), 7.31 - 7.21 (m, 1H), 3.96 (s, 3H), 3.84 - 3.55 (m, 3H)

The Examples in Table 1 were prepared using procedures similar to those used to prepare the Examples shown above. Examples 2, 3, 6, 7, 9-13, 15-20, 22-28, 31, 33-37, 39, 40, 42-49, 72, 74-77 are Reference Examples.

**Table 1**

| Ex. No. | Structure | Mol Wt | LCMS *m*/*z* observed | HPLC *t*_{R} (min) | HPLC Method |
|---|---|---|---|---|---|
| 1 | 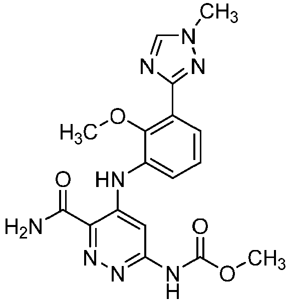 | 398.38 | 399.3 | 0.75 | QC-ACN-TFA-XB |
| 2 | 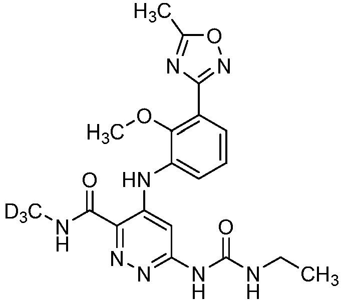 | 429.46 | 430.1 | 1.11 | QC-ACN-TFA-XB |
| 3 | 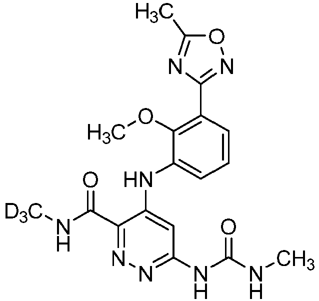 | 415.43 | 415.9 | 1.41 | QC-ACN-AA-XB |
| 4 | 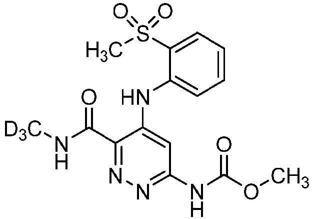 | 382.41 | 383.0 | 1.04 | QC-ACN-AA-XB |
| 5 | 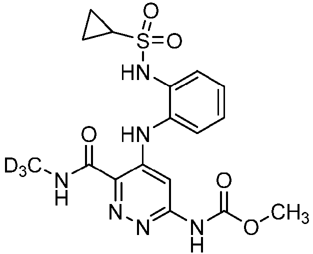 | 423.46 | 424.0 | 1.21 | QC-ACN-AA-XB |
| 6 | 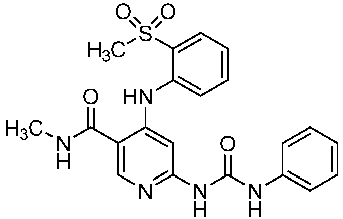 | 439.49 | 440.1 | 1.16 | QC-ACN-TFA-XB |
| 7 | 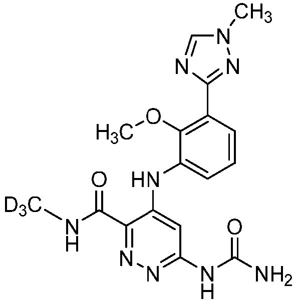 | 400.42 | 401.3 | 0.69 | QC-ACN-TFA-XB |
| 8 | 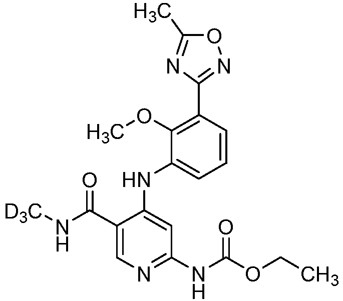 | 429.45 | 430.1 | 0.73 | Method B |
| 9 | 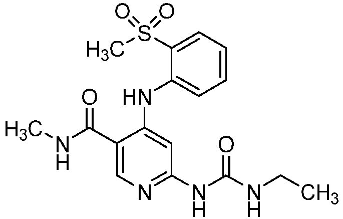 | 391.45 | 392.0 | 0.93 | QC-ACN-TFA-XB |
| 10 | 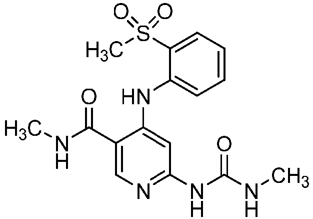 | 377.42 | 378.0 | 0.97 | QC-ACN-AA-XB |
| 11 | 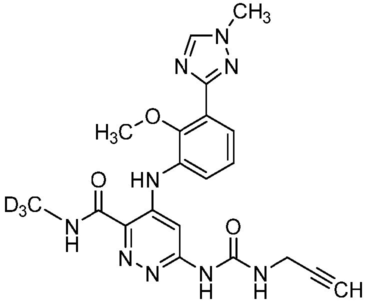 | 438.47 | 439.4 | 1.05 | QC-ACN-AA-XB |
| 12 | 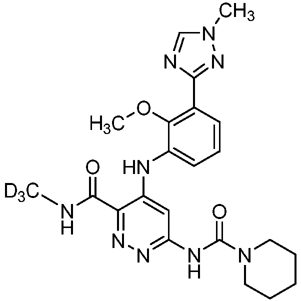 | 468.54 | 469.2 | 1.04 | QC-ACN-TFA-XB |
| 13 | 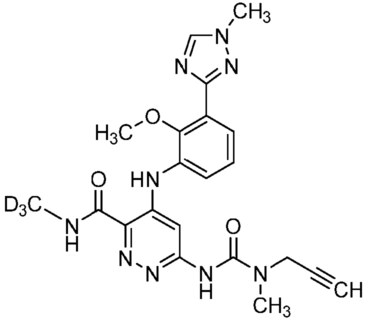 | 452.49 | 453.0 | 1.07 | QC-ACN-AA-XB |
| 14 | 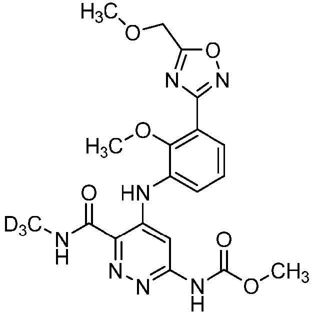 | 446.44 | 446.9 | 1.37 | QC-ACN-TFA-XB |
| 15 | 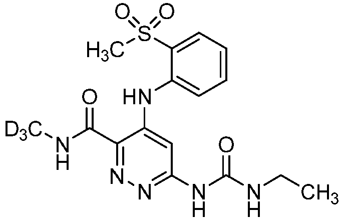 | 395.45 | 396.2 | 0.83 | QC-ACN-TFA-XB |
| 16 | 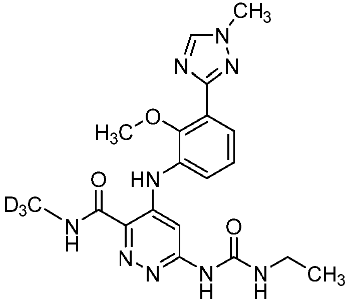 | 428.47 | 429.2 | 0.90 | QC-ACN-TFA-XB |
| 17 | 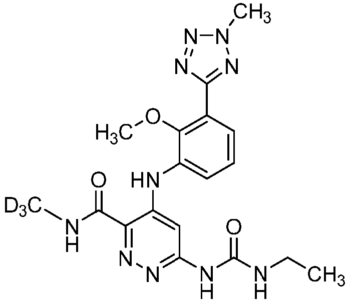 | 429.46 | 430.2 | 1.05 | QC-ACN-TFA-XB |
| 18 | 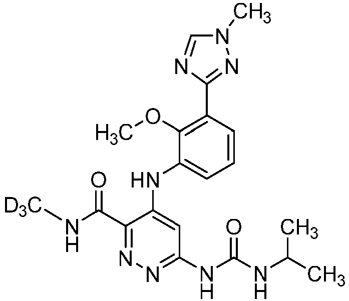 | 442.50 | 443.2 | 1.00 | QC-ACN-TFA-XB |
| 19 | 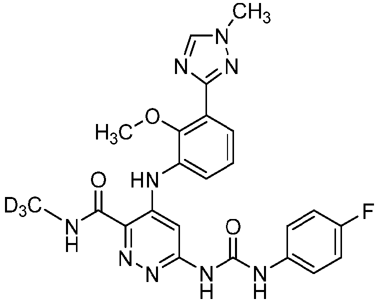 | 494.51 | 495.2 | 1.53 | QC-ACN-AA-XB |
| 20 | 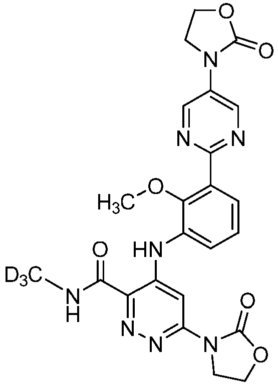 | 509.50 | 510.2 | 1.11 | QC-ACN-TFA-XB |
| 21 | 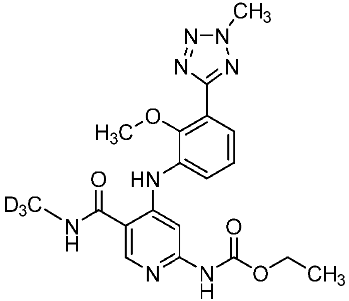 | 429.46 | 430.3 | 1.06 | QC-ACN-TFA-XB |
| 22 | 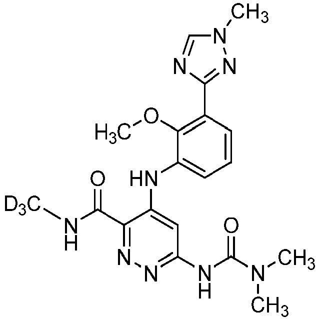 | 428.47 | 429.2 | 0.74 | QC-ACN-TFA-XB |
| 23 | 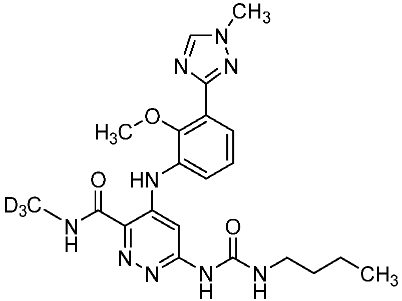 | 456.53 | 457.2 | 1.41 | QC-ACN-AA-XB |
| 24 | 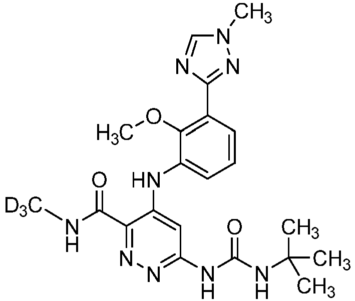 | 456.53 | 457.3 | 1.04 | QC-ACN-TFA-XB |
| 25 | 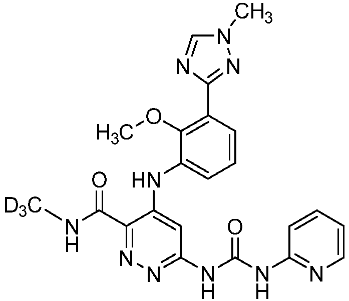 | 477.50 | 478.1 | 0.98 | QC-ACN-TFA-XB |
| 26 | 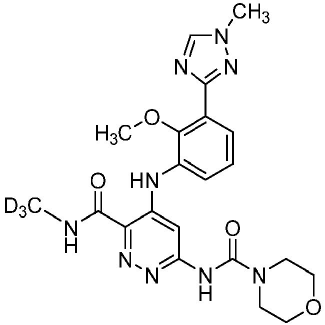 | 470.51 | 471.4 | 0.96 | QC-ACN-AA-XB |
| 27 | 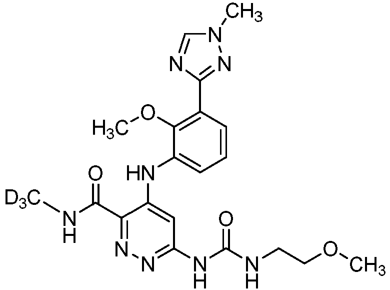 | 458.50 | 459.2 | 0.87 | QC-ACN-TFA-XB |
| 28 | 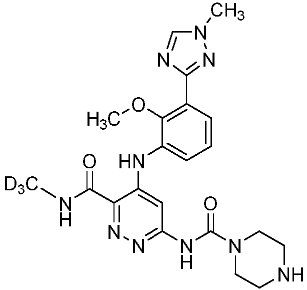 | 469.52 | 470.1 | 0.70 | QC-ACN-TFA-XB |
| 29 | 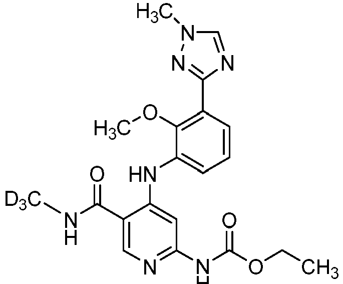 | 428.47 | 429.1 | 0.92 | QC-ACN-TFA-XB |
| 30 | 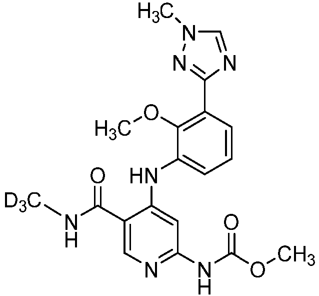 | 414.44 | 415.2 | 0.77 | QC-ACN-TFA-XB |
| 31 | 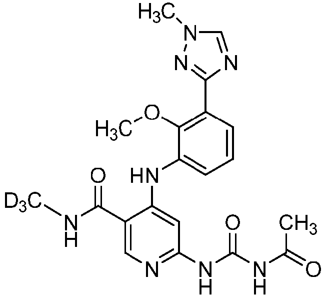 | 441.47 | 442.1 | 1.00 | QC-ACN-AA-XB |
| 32 | 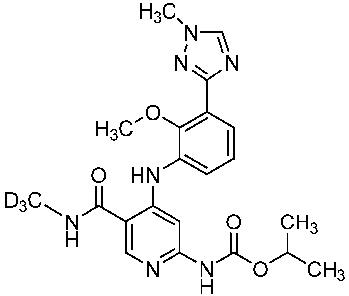 | 442.49 | 443.2 | 1.19 | QC-ACN-AA-XB |
| 33 | 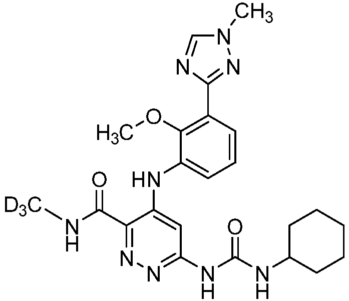 | 482.56 | 483.2 | 1.22 | QC-ACN-TFA-XB |

| Ex. No. | Structure | Mol Wt | LCMS *m*/*z+* observed | HPLC *t*_{R} (min) | HPLC Method |
|---|---|---|---|---|---|
| 34 | 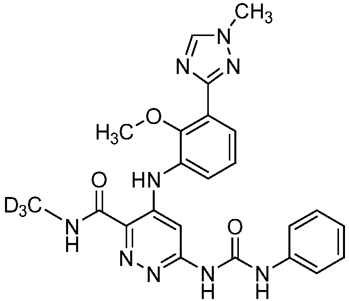 | 476.52 | 477.4 | 1.39 | QC-ACN-AA-XB |
| 35 | 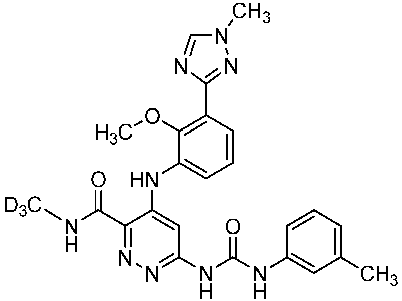 | 490.54 | 491.4 | 1.52 | QC-ACN-AA-XB |
| 36 | 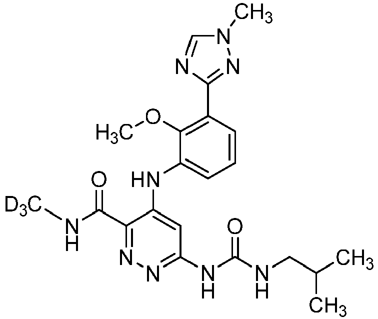 | 456.53 | 457.3 | 1.09 | QC-ACN-TFA-XB |

| Ex. No. | Structure | Mol Wt | LCMS *m*/*z* observed | HPLC *t*_{R} (min) | HPLC Method |
|---|---|---|---|---|---|
| 37 | 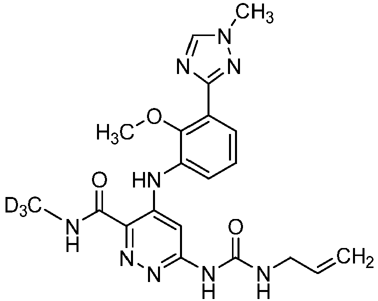 | 440.48 | 441.2 | 1.17 | QC-ACN-AA-XB |
| 38 | 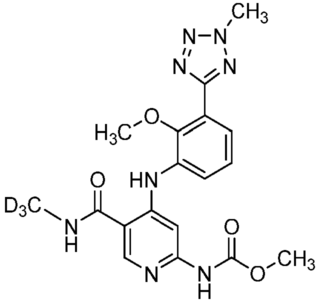 | 415.43 | 416.3 | 1.16 | QC-ACN-AA-XB |
| 39 | 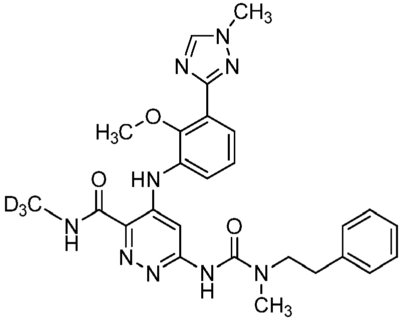 | 518.60 | 519.3 | 1.20 | QC-ACN-TFA-XB |
| 40 | 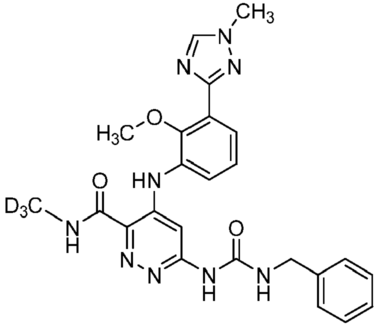 | 490.54 | 491.2 | 1.42 | QC-ACN-AA-XB |
| 41 | 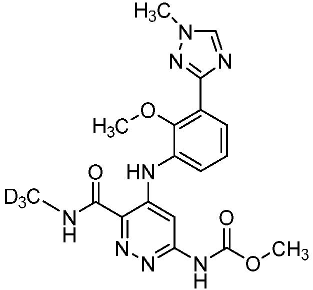 | 415.43 | 416.1 | 1.06 | QC-ACN-AA-XB |
| 42 | 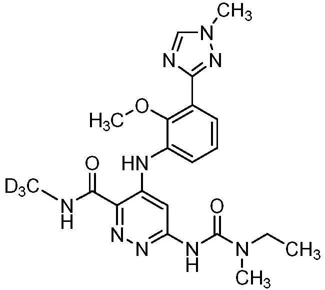 | 442.50 | 443.3 | 1.08 | QC-ACN-AA-XB |
| 43 | 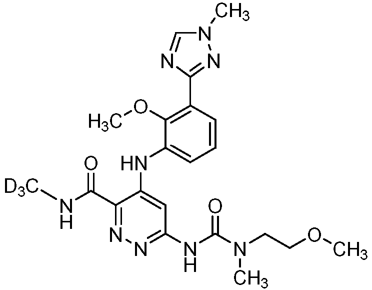 | 472.52 | 473.4 | 0.86 | QC-ACN-TFA-XB |
| 44 | 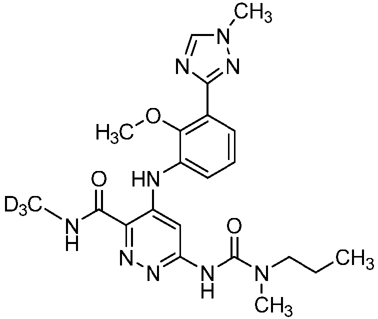 | 456.53 | 457.2 | 1.01 | QC-ACN-TFA-XB |
| 45 | 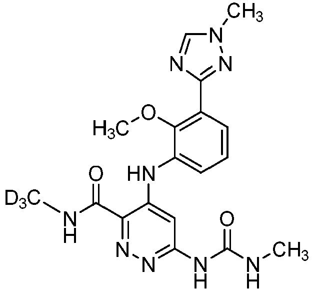 | 414.44 | 415.2 | 0.93 | QC-ACN-AA-XB |
| 46 | 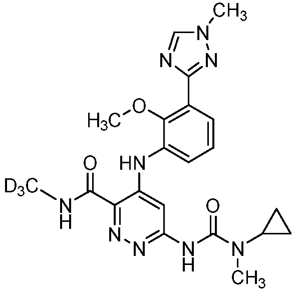 | 454.51 | 455.4 | 1.16 | QC-ACN-AA-XB |
| 47 | 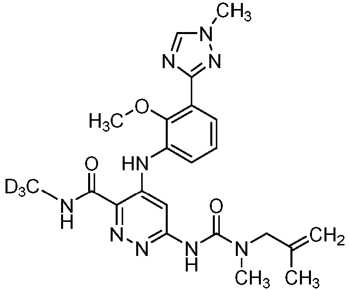 | 468.54 | 469.1 | 1.06 | QC-ACN-TFA-XB |
| 48 | 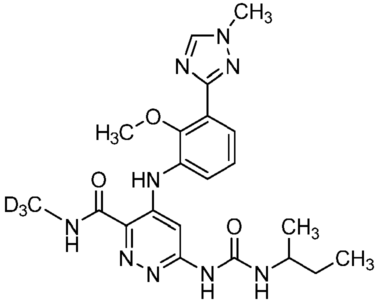 | 456.53 | 457.1 | 1.06 | QC-ACN-TFA-XB |
| 49 | 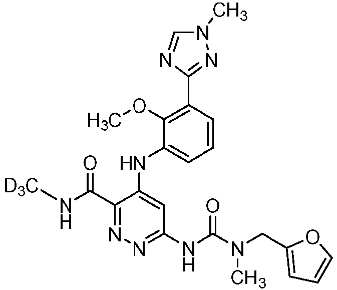 | 494.53 | 495.2 | 1.03 | QC-ACN-TFA-XB |
| 50 | 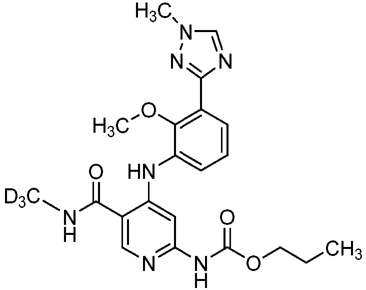 | 442.49 | 443.3 | 0.96 | QC-ACN-TFA-XB |
| 51 | 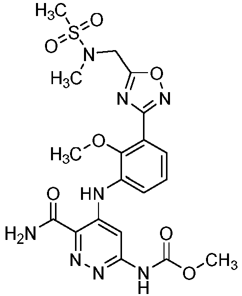 | 506.49 | 507.0 | 1.16 | QC-ACN-AA-XB |
| 52 | 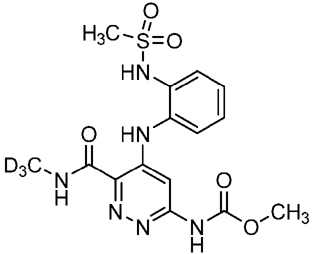 | 397.42 | 398.3 | 0.79 | QC-ACN-TFA-XB |
| 53 | 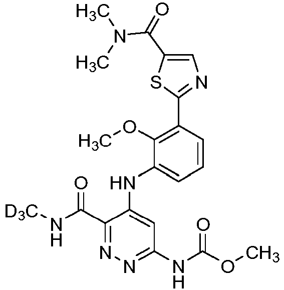 | 488.54 | 489.1 | 1.42 | QC-ACN-AA-XB |
| 54 | 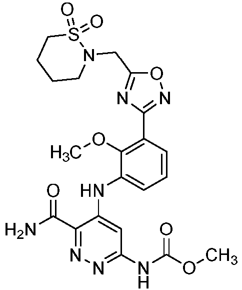 | 532.53 | 533.1 | 1.36 | QC-ACN-AA-XB |
| 55 | 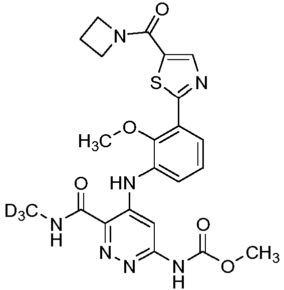 | 500.55 | 501.1 | 1.62 | QC-ACN-AA-XB |
| 56 | 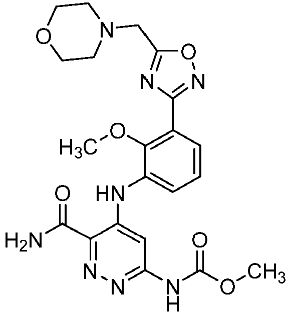 | 484.47 | 485.3 | 1.19 | QC-ACN-AA-XB |
| 57 | 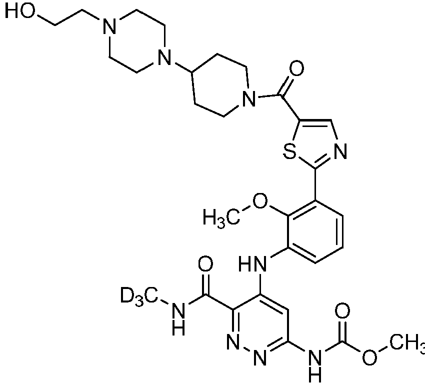 | 656.78 | 657.3 | 1.08 | QC-ACN-AA-XB |
| 58 | 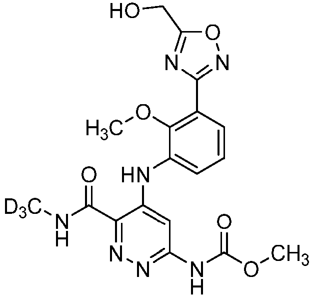 | 432.41 | 433.2 | 1.10 | QC-ACN-AA-XB |
| 59 | 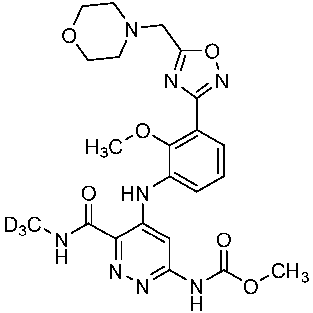 | 501.52 | 502.3 | 0.85 | QC-ACN-TFA-XB |
| 60 | 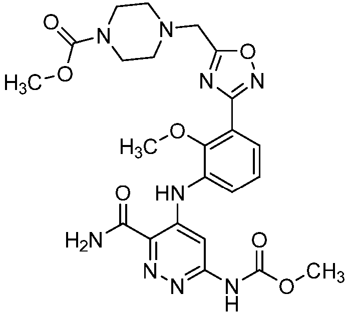 | 541.53 | 542.1 | 1.31 | QC-ACN-AA-XB |
| 61 | 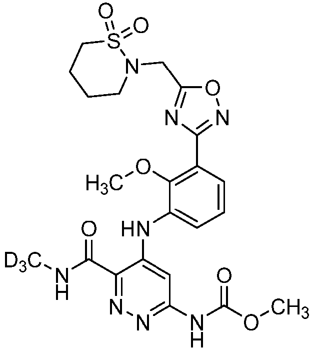 | 549.58 | 550.2 | 1.37 | QC-ACN-TFA-XB |
| 62 | 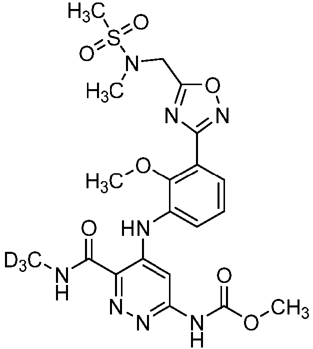 | 523.54 | 524.2 | 1.33 | QC-ACN-AA-XB |
| 63 | 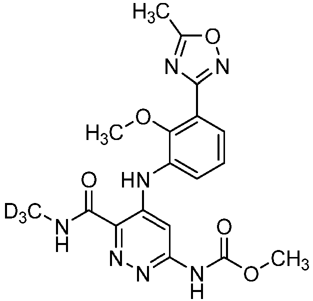 | 416.41 | 417.2 | 1.35 | QC-ACN-AA-XB |
| 64 | 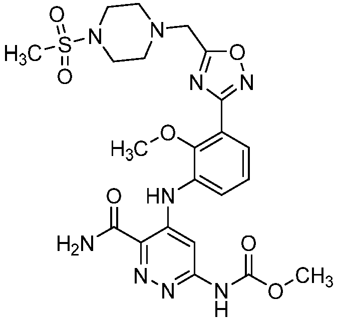 | 561.57 | 562.3 | 1.22 | QC-ACN-AA-XB |
| 65 | 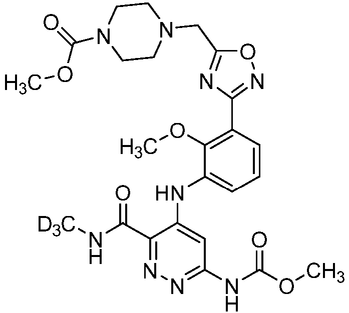 | 558.57 | 559.2 | 1.43 | QC-ACN-AA-XB |
| 66 | 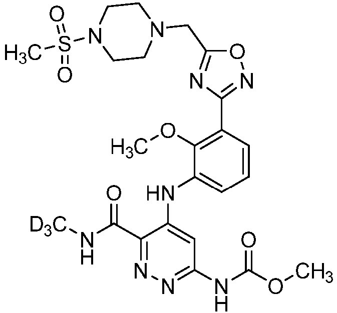 | 578.62 | 579.2 | 1.33 | QC-ACN-AA-XB |
| 67 | 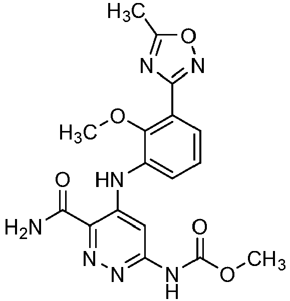 | 399.37 | 400.2 | 1.25 | QC-ACN-TFA-XB |
| 68 | 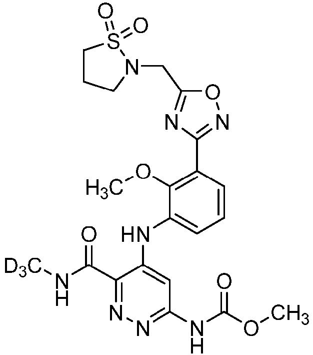 | 535.55 | 536.1 | 1.27 | QC-ACN-AA-XB |
| 69 | 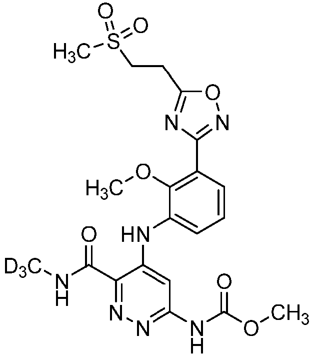 | 508.52 | 509.2 | 1.02 | QC-ACN-AA-XB |
| 70 | 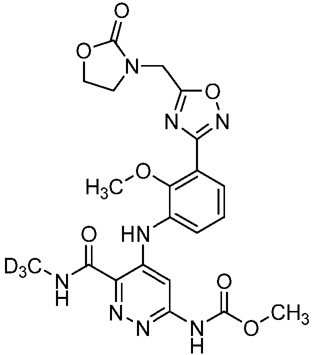 | 501.47 | 502.2 | 0.97 | QC-ACN-AA-XB |
| 71 | 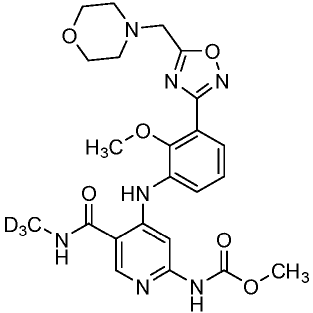 | 500.53 | 501.3 | 1.32 | QC-ACN-AA-XB |
| 72 | 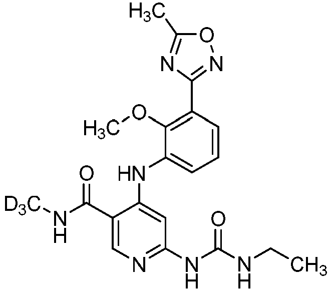 | 428.47 | 429.0 | 1.30 | QC-ACN-AA-XB |
| 73 | 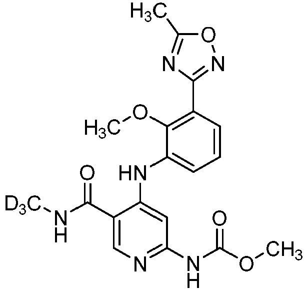 | 415.42 | 416.2 | 1.31 | QC-ACN-AA-XB |
| 74 | 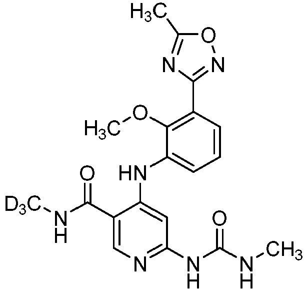 | 414.44 | 415.3 | 1.22 | QC-ACN-AA-XB |
| 75 | 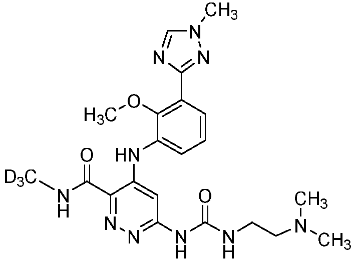 | 471.54 | 472.2 | 0.85 | QC-ACN-AA-XB |
| 76 | 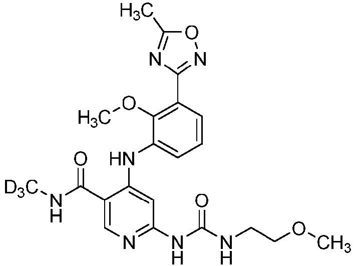 | 458.49 | 459.2 | 1.26 | QC-ACN-AA-XB |
| 77 | 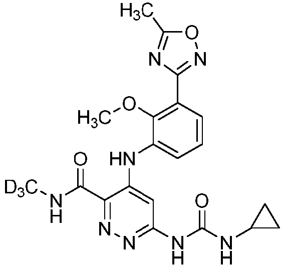 | 441.47 | 442.1 | 1.13 | QC-ACN-TFA-XB |

### BIOLOGICAL ASSAYS

The following assay is used to show the activity for compounds of the invention.

### IFNα-Induced STAT Phosphorylation in Human Whole Blood

After an hour long incubation with compound, human whole blood (drawn with either EDTA or ACD-A as anti-coagulant) was stimulated with 1000 U/mL recombinant human IFNα A/D (R&D Systems 11200-2) for 15 min. The stimulation was stopped by adding Fix/Lyse buffer (BD 558049). Cells were stained with a CD3 FITC antibody (BD 555916), washed, and permeabilized on ice using Perm III buffer (BD 558050). Cells were then stained with an Alexa-Fluor 647 pSTAT5 (pY694) antibody (BD 612599) for 30 min prior to analysis on the FACS Canto II. The amount of pSTATS expression was quantitated by median fluorescence intensity after gating on the CD3 positive population.

### IFNα-Induced STAT Phosphorylation in Human Whole Blood Inhibition Data

| Example No. | Human WB IFNα-Induced Stat Phosph. (IC₅₀, µM) |
|---|---|
| 1 | 0.07 |
| 2 | 0.11 |
| 3 | 0.19 |
| 4 | >10 |
| 5 | 0.75 |
| 6 | >10 |
| 7 | 0.32 |
| 8 | 0.30 |
| 9 | 1.10 |
| 10 | 3.69 |
| 11 | 0.06 |
| 12 | 0.61 |
| 13 | ND |
| 14 | 1.10 |
| 15 | 0.52 |
| 16 | 0.02 |
| 17 | ND |
| 18 | 0.15 |
| 19 | ND |
| 20 | >10 |
| 21 | 0.42 |
| 22 | 0.22 |
| 23 | 0.15 |
| 24 | 0.23 |
| 25 | 1.18 |
| 26 | 1.18 |
| 27 | 0.14 |
| 28 | >10 |
| 29 | 0.11 |
| 30 | 0.10 |
| 31 | ND |
| 32 | 0.20 |
| 33 | 0.12 |
| 34 | 0.52 |
| 35 | 0.50 |
| 36 | 0.15 |
| 37 | 0.06 |
| 38 | 0.13 |
| 39 | 2.46 |
| 40 | 0.29 |
| 41 | 0.21 |
| 42 | 0.35 |
| 43 | 0.57 |
| 44 | 0.33 |
| 45 | 0.08 |
| 46 | 2.21 |
| 47 | 0.43 |
| 48 | 0.20 |
| 49 | 0.19 |
| 50 | 0.15 |
| 51 | 0.09 |
| 52 | >10 |
| 53 | 0.84 |
| 54 | 0.03 |
| 55 | 0.76 |
| 56 | 0.11 |
| 57 | 0.25 |
| 58 | 1.86 |
| 59 | 0.29 |
| 60 | 0.14 |
| 61 | 0.06 |
| 62 | 0.10 |
| 63 | 0.97 |
| 64 | 0.10 |
| 65 | 0.33 |
| 66 | 0.13 |
| 67 | 0.27 |
| 68 | 0.20 |
| 69 | 1.03 |
| 70 | 1.15 |
| 71 | 0.30 |
| 72 | 0.14 |
| 73 | 0.77 |
| 74 | 0.21 |
| 75 | 0.47 |
| 76 | 0.16 |
| 77 | 0.21 |

| | |
|---|---|
| ND - no data available | |

## Claims

1. A compound of formula I: 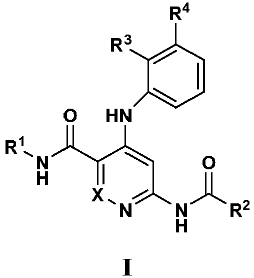 wherein
X is N or CH;
R¹ is H, CD₃, C₁₋₃ alkyl or C₃₋₆ cycloalkyl;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 1 or 2;
r is 0, 1, 2, 3, 4 or 5;
or a stereoisomer or pharmaceutically acceptable salt thereof.

2. The compound according to claim 1 of the formula 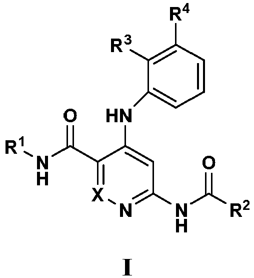 wherein
X is N or CH;
R¹ is H or CD₃;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 1 or 2;
r is 0, 1, 2, 3, 4 or 5;
or a stereoisomer or pharmaceutically acceptable salt thereof.

3. The compound according to claim 1 of the formula 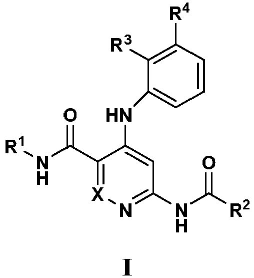 wherein
X is N or CH;
R¹ is H or CD₃;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1, 2, 3, 4 or 5;
or a stereoisomer or pharmaceutically acceptable salt thereof.

4. The compound according to claim 3 of the formula 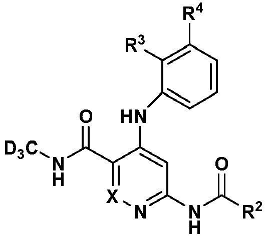 wherein
X is N or CH;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1, 2, 3, 4 or 5;
or a stereoisomer or pharmaceutically acceptable salt thereof.

5. The compound according to claim 4 of the formula 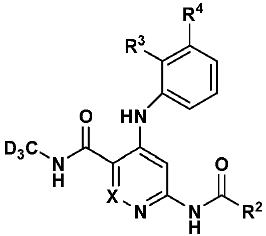 wherein
X is N or CH;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1 or 2;
or a stereoisomer or pharmaceutically acceptable salt thereof.

6. The compound according to claim 5 of the formula 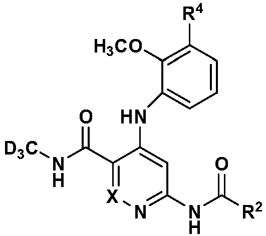 wherein
X is N or CH;
R² is C₁₋₆ alkoxy;
R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1 or 2;
or a stereoisomer or pharmaceutically acceptable salt thereof.

7. The compound according to claim 6 of the formula 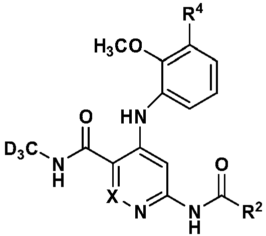 wherein
X is N or CH;
R² is C₁₋₆ alkoxy;
R⁴ is absent or a triazole, thiazole, tetrazole or oxadiazole group each of which is substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 2;
r is 0, 1 or 2;
or a stereoisomer or pharmaceutically acceptable salt thereof.

8. The compound according to claim 1 of the formula 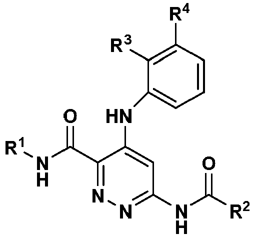 wherein
R' is H, CD₃, C₁₋₃ alkyl or C₃₋₆ cycloalkyl;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 1 or 2;
r is 0, 1, 2, 3, 4 or 5;
or a stereoisomer or pharmaceutically acceptable salt thereof.

9. The compound according to claim 1 of the formula 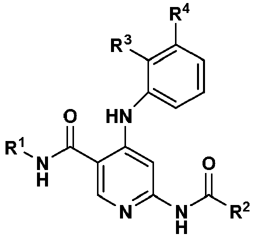 wherein
R' is H, CD₃, C₁₋₃ alkyl or C₃₋₆ cycloalkyl;
R² is C₁₋₆ alkoxy;
R³ is C₁₋₃ alkyl, C₁₋₃ alkoxy, -NHS(O)ₚR^{a} or -S(O)ₚR^{a};R⁴ is absent or a 5-7 membered heterocycle substituted with 0-3 R⁵;
R⁵ is H, halogen, C₁₋₃ alkyl, C₁₋₃ alkyl C₁₋₃ alkoxy, --(CH₂)ᵣ 5-8 membered heterocycle substituted with 0-2 R^{5a} or -CO-3-8 membered heterocycle;
R^{5a} is SO₂- C₁₋₆ alkyl or COO- C₁₋₆ alkyl;
R^{a} is C₁₋₃ alkyl or C₃₋₆ cycloalkyl;
p is 1 or 2;
r is 0, 1, 2, 3, 4 or 5;
or a stereoisomer or pharmaceutically acceptable salt thereof.

10. The compound according to claim 1 which is one of the following compounds; 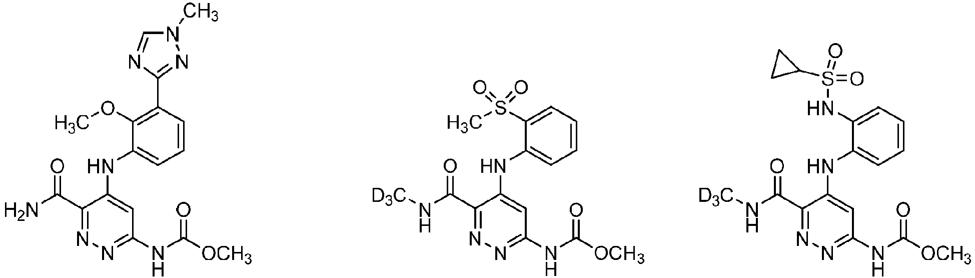 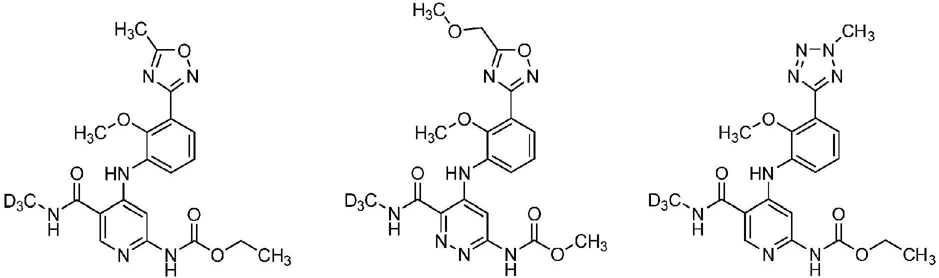 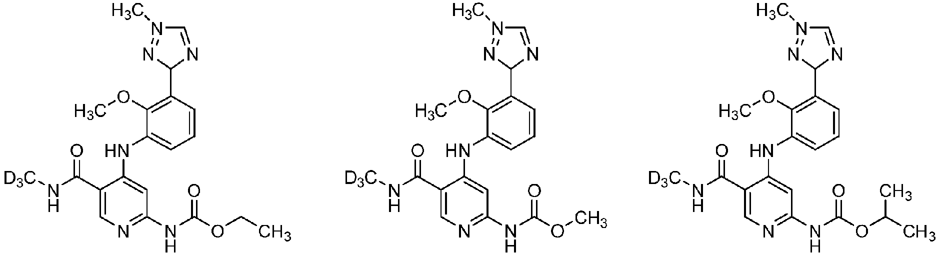 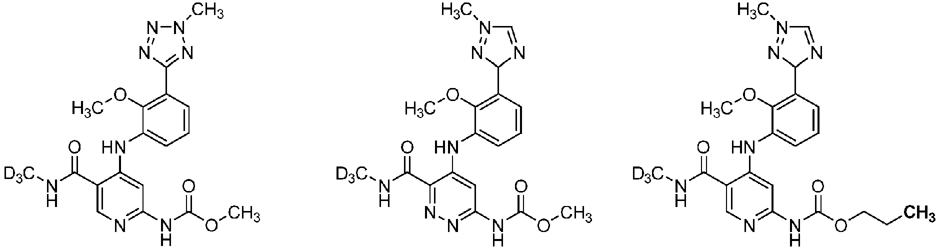 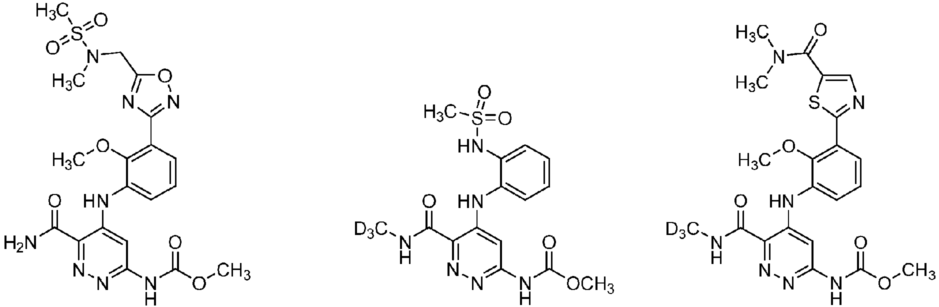 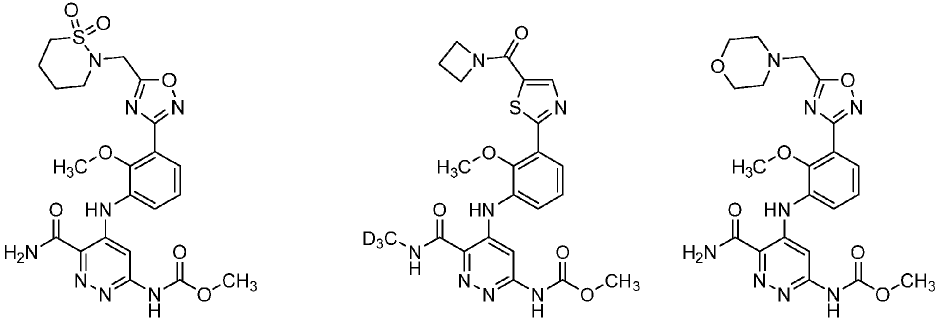 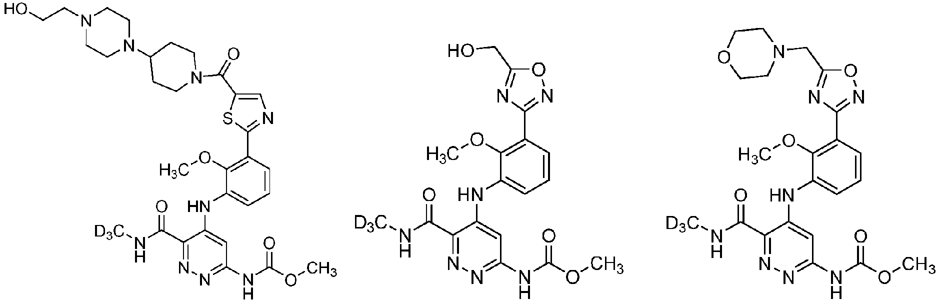 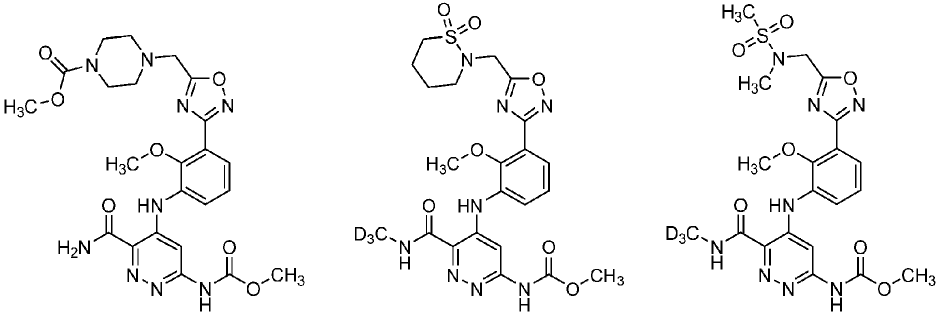 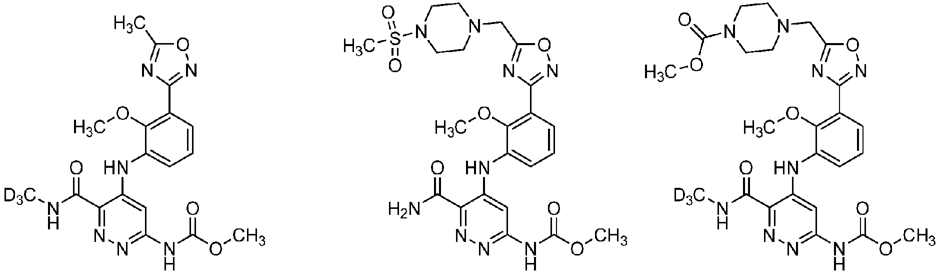 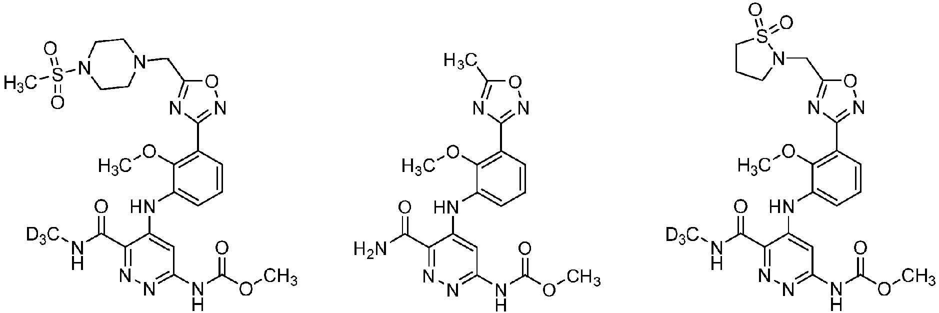 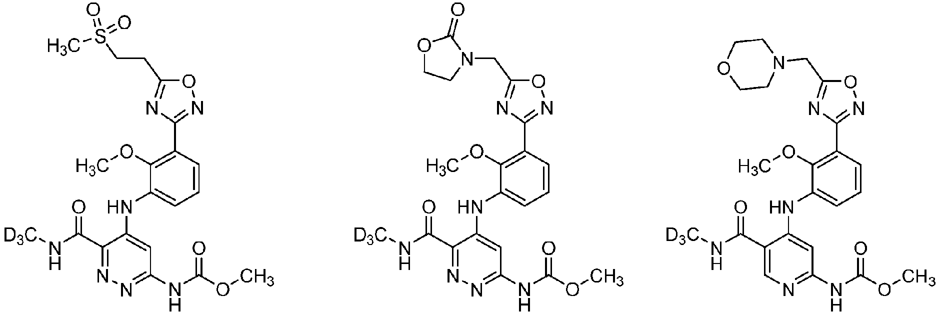 and

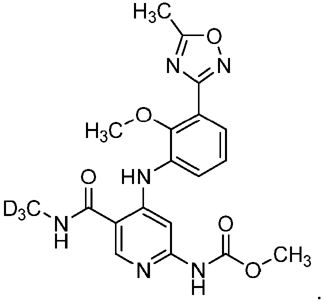

11. A pharmaceutical composition comprising one or more compounds according to any one of claims 1 to 10 and a pharmaceutically acceptable carrier or diluent.

12. A compound according to any one of claims 1 to 10 for use in therapy.

13. A compound according to any one of claims 1 to 10 for use in a method of treating a disease, wherein the disease is an inflammatory or autoimmune disease.

14. The compound for use of claim 13 wherein the inflammatory or autoimmune disease is multiple sclerosis, rheumatoid arthritis, ankylosing spondylitis, inflammatory bowel disease, systemic lupus erythematosus, psoriasis, psoriatic arthritis, Crohn's Disease, Sjögren's syndrome or scleroderma.

## Patentansprüche

1. Verbindung mit der Formel I: 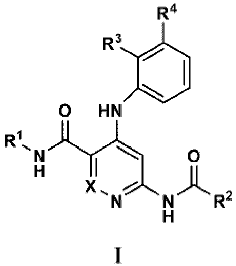 wobei
X N oder CH ist;
R¹ H, CD₃, C₁₋₃-Alkyl oder C₃₋₆-Cycloalkyl ist;
R² C₁₋₆-Alkoxy ist;
R³ C₁₋₃-Alkyl, C₁₋₃-Alkoxy, -NHS(O)ₚR^{a} oder -S(O)ₚR^{a} ist;
R⁴ nicht vorhanden oder ein 5-7-gliedriger Heterocyclus, substituiert mit 0-3 R⁵, ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 1 oder 2 ist;
r 0, 1, 2, 3, 4 oder 5 ist;
oder ein Stereoisomer oder pharmazeutisch verträgliches Salz davon.

2. Verbindung nach Anspruch 1 mit der Formel 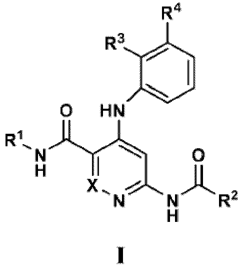 wobei
X N oder CH ist;
R¹ H oder CD₃ ist;
R² C₁₋₆-Alkoxy ist;
R³ C₁₋₃-Alkyl, C₁₋₃-Alkoxy, -NHS(O)ₚR^{a} oder -S(O)ₚR^{a} ist;
R⁴ nicht vorhanden oder ein 5-7-gliedriger Heterocyclus, substituiert mit 0-3 R⁵, ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 1 oder 2 ist;
r 0, 1, 2, 3, 4 oder 5 ist;
oder ein Stereoisomer oder pharmazeutisch verträgliches Salz davon.

3. Verbindung nach Anspruch 1 mit der Formel 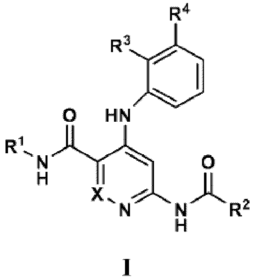 wobei
X N oder CH ist;
R¹ H oder CD₃ ist;
R² C₁₋₆-Alkoxy ist;
R³ C₁₋₃-Alkyl, C₁₋₃-Alkoxy, -NHS(O)ₚR^{a} oder -S(O)ₚR^{a} ist;
R⁴ nicht vorhanden oder ein 5-7-gliedriger Heterocyclus, substituiert mit 0-3 R⁵, ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 2 ist;
r 0, 1, 2, 3, 4 oder 5 ist;
oder ein Stereoisomer oder pharmazeutisch verträgliches Salz davon.

4. Verbindung nach Anspruch 3 mit der Formel 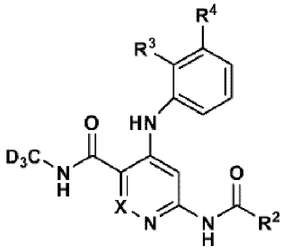 wobei
X N oder CH ist;
R² C₁₋₆-Alkoxy ist;
R³ C₁₋₃-Alkyl, C₁₋₃-Alkoxy, -NHS(O)ₚR^{a} oder -S(O)ₚR^{a} ist;
R⁴ nicht vorhanden oder ein 5-7-gliedriger Heterocyclus, substituiert mit 0-3 R⁵, ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 2 ist;
r 0, 1, 2, 3, 4 oder 5 ist;
oder ein Stereoisomer oder pharmazeutisch verträgliches Salz davon.

5. Verbindung nach Anspruch 4 mit der Formel 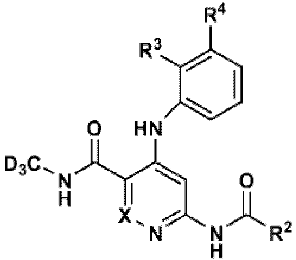 wobei
X N oder CH ist;
R² C₁₋₆-Alkoxy ist;
R³ C₁₋₃-Alkyl, C₁₋₃-Alkoxy, -NHS(O)ₚR^{a} oder -S(O)ₚR^{a} ist;
R⁴ nicht vorhanden oder ein 5-7-gliedriger Heterocyclus, substituiert mit 0 bis 3 R⁵, ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 2 ist;
r 0, 1 oder 2 ist;
oder ein Stereoisomer oder pharmazeutisch verträgliches Salz davon.

6. Verbindung nach Anspruch 5 mit der Formel 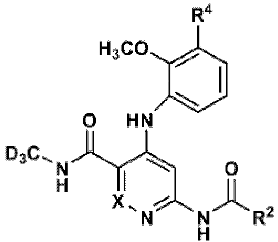 wobei
X N oder CH ist;
R² C₁₋₆-Alkoxy ist;
R⁴ nicht vorhanden oder ein 5-7-gliedriger Heterocyclus, substituiert mit 0-3 R⁵, ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 2 ist;
r 0, 1 oder 2 ist;
oder ein Stereoisomer oder pharmazeutisch verträgliches Salz davon.

7. Verbindung nach Anspruch 6 mit der Formel 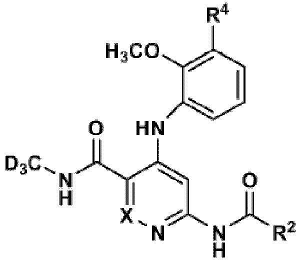 wobei
X N oder CH ist;
R² C₁₋₆-Alkoxy ist;
R⁴ nicht vorhanden oder eine Triazol-, Thiazol-, Tetrazol- oder Oxadiazolgruppe ist, von denen jede mit 0-3 R⁵ substituiert ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 2 ist;
r 0, 1 oder 2 ist;
oder ein Stereoisomer oder pharmazeutisch verträgliches Salz davon.

8. Verbindung nach Anspruch 1 mit der Formel 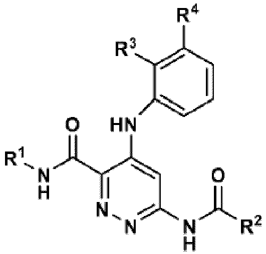 wobei
R¹ H, CD₃, C₁₋₃-Alkyl oder C₃₋₆-Cycloalkyl ist;
R² C₁₋₆-Alkoxy ist;
R³ C₁₋₃-Alkyl, C₁₋₃-Alkoxy, -NHS(O)ₚR^{a} oder -S(O)ₚR^{a} ist;
R⁴ nicht vorhanden oder ein 5-7-gliedriger Heterocyclus, substituiert mit 0-3 R⁵, ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 1 oder 2 ist;
r 0, 1, 2, 3, 4 oder 5 ist;
oder ein Stereoisomer oder pharmazeutisch verträgliches Salz davon.

9. Verbindung nach Anspruch 1 mit der Formel 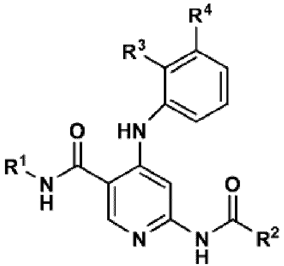 wobei
R¹ H, CD₃, C₁₋₃-Alkyl oder C₃₋₆-Cycloalkyl ist;
R² C₁₋₆-Alkoxy ist;
R³ C₁₋₃-Alkyl, C₁₋₃-Alkoxy, -NHS(O)ₚR^{a} oder -S(O)ₚR^{a} ist;
R⁴ nicht vorhanden oder ein 5-7-gliedriger Heterocyclus, substituiert mit 0-3 R⁵, ist;
R⁵ H, Halogen, C₁₋₃-Alkyl, C₁₋₃-Alkyl-C₁₋₃-alkoxy, -(CH₂)ᵣ-5-8-gliedriger Heterocyclus, substituiert mit 0-2 R^{5a}, oder -CO-3-8-gliedriger Heterocyclus ist;
R^{5a} SO₂-C₁₋₆-Alkyl oder COO-C₁₋₆-Alkyl ist;
R^{a} C₁₋₃-Alkyl oder C₃₋₆-Cycloalkyl ist;
p 1 oder 2 ist;
r 0, 1, 2, 3, 4 oder 5 ist;
oder ein Stereoisomer oder pharmazeutisch verträgliches Salz davon.

10. Verbindung nach Anspruch 1, die eine der folgenden Verbindungen ist: 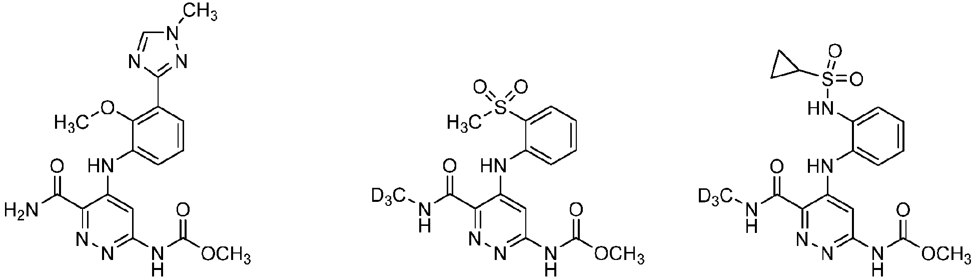 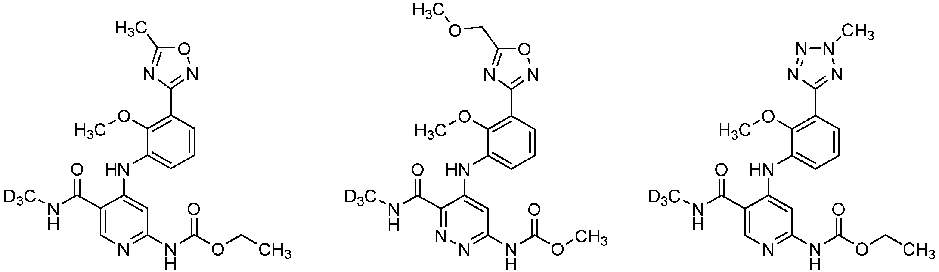 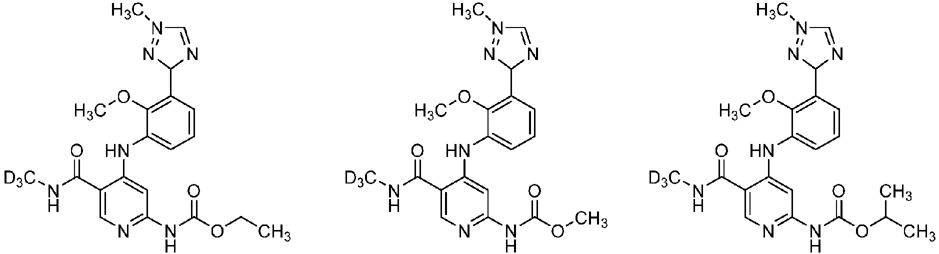 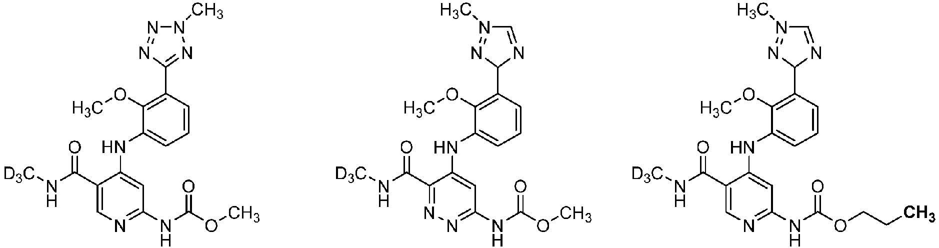 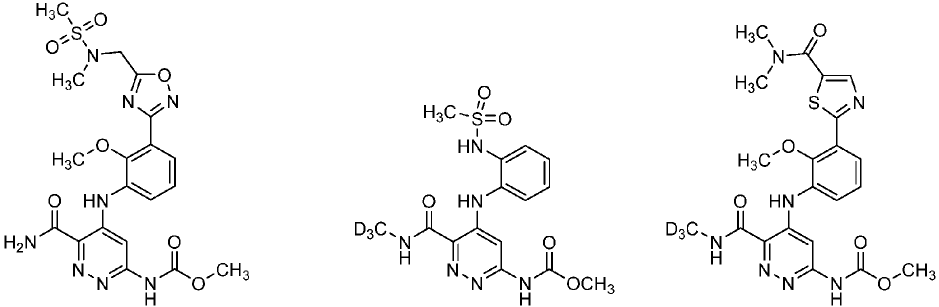 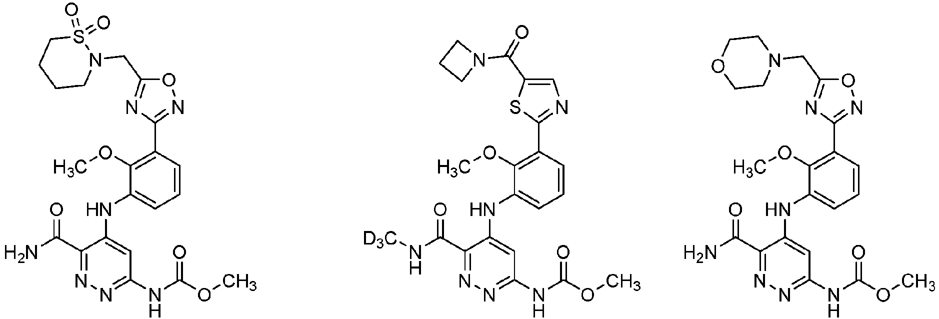 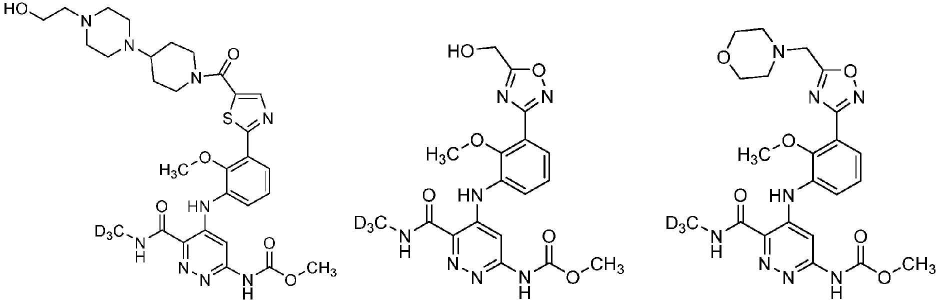 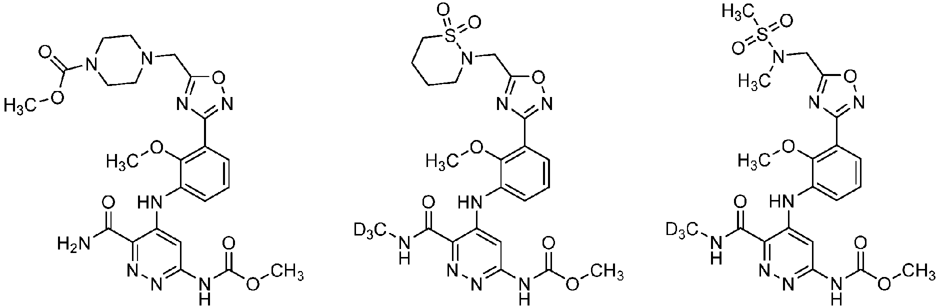 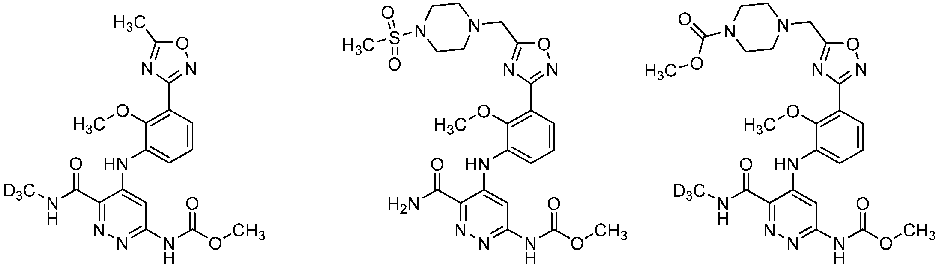 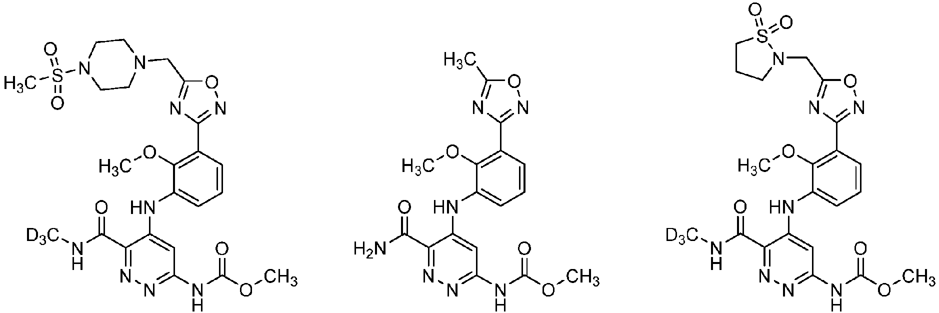 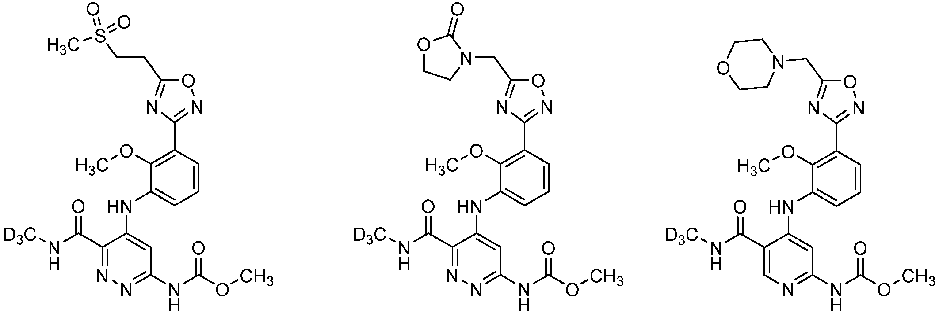 und

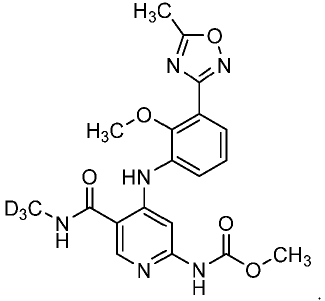

11. Pharmazeutische Zusammensetzung, umfassend eine oder mehrere Verbindungen nach einem der Ansprüche 1 bis 10 und ein pharmazeutisch verträgliches Träger- oder Verdünnungsmittel.

12. Verbindung nach einem der Ansprüche 1 bis 10 zur Verwendung in der Therapie.

13. Verbindung nach einem der Ansprüche 1 bis 10 zur Verwendung in einem Verfahren zur Behandlung einer Erkrankung, wobei die Erkrankung eine Entzündungs- oder Autoimmunerkrankung ist.

14. Verbindung zur Verwendung nach Anspruch 13, wobei die Entzündungs- oder Autoimmunerkrankung Multiple Sklerose, rheumatoide Arthritis, Morbus Bechterew, entzündliche Darmerkrankung, systemischer Lupus erythematodes, Psoriasis, Psoriasis-Arthritis, Morbus Crohn, Sjögren-Syndrom oder Sklerodermie ist.

## Revendications

1. Composé de formule I : 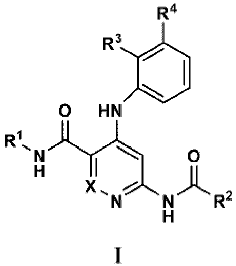 où :
X est N ou CH ;
R¹ est H, CD₃, un C₁₋₃ alkyle ou un C₃₋₆ cycloalkyle ;
R² est un C₁₋₆ alcoxy ;
R³ est un C₁₋₃ alkyle, un C₁₋₃ alcoxy, -NHS(O)ₚR^{a} ou -S(O)ₚR^{a} ;
R⁴ est absent ou un hétérocycle à 5-7 chaînons substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 1 ou 2 ;
r est 0, 1, 2, 3, 4 ou 5 ;
ou un stéréoisomère ou sel pharmaceutiquement acceptable de celui-ci.

2. Composé selon la revendication 1 de formule : 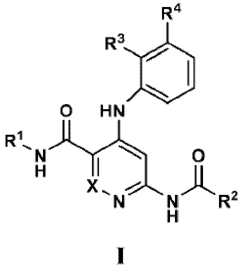 où :
X est N ou CH ;
R¹ est H ou CD₃ ;
R² est un C₁₋₆ alcoxy ;
R³ est un C₁₋₃ alkyle, un C₁₋₃ alcoxy, -NHS(O)ₚR^{a} ou -S(O)ₚR^{a} ;
R⁴ est absent ou un hétérocycle à 5-7 chaînons substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 1 ou 2 ;
r est 0, 1, 2, 3, 4 ou 5 ;
ou un stéréoisomère ou sel pharmaceutiquement acceptable de celui-ci.

3. Composé selon la revendication 1 de formule : 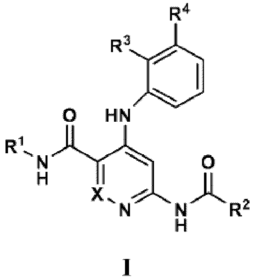 où :
X est N ou CH ;
R¹ est H ou CD₃ ;
R² est un C₁₋₆ alcoxy ;
R³ est un C₁₋₃ alkyle, un C₁₋₃ alcoxy, -NHS(O)ₚR^{a} ou -S(O)ₚR^{a} ;
R⁴ est absent ou un hétérocycle à 5-7 chaînons substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 2 ;
r est 0, 1, 2, 3, 4 ou 5 ;
ou un stéréoisomère ou sel pharmaceutiquement acceptable de celui-ci.

4. Composé selon la revendication 3 de formule : 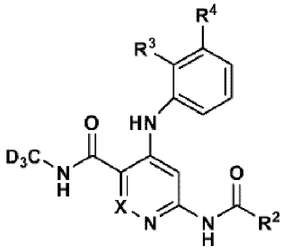 où :
X est N ou CH ;
R² est un C₁₋₆ alcoxy ;
R³ est un C₁₋₃ alkyle, un C₁₋₃ alcoxy, -NHS(O)ₚR^{a} ou -S(O)ₚR^{a} ;
R⁴ est absent ou un hétérocycle à 5-7 chaînons substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 2 ;
r est 0, 1, 2, 3, 4 ou 5 ;
ou un stéréoisomère ou sel pharmaceutiquement acceptable de celui-ci.

5. Composé selon la revendication 4 de formule : 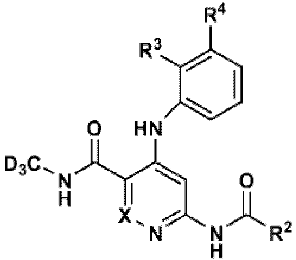 où :
X est N ou CH ;
R² est un C₁₋₆ alcoxy ;
R³ est un C₁₋₃ alkyle, un C₁₋₃ alcoxy, -NHS(O)ₚR^{a} ou -S(O)ₚR^{a} ;
R⁴ est absent ou un hétérocycle à 5-7 chaînons substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 2 ;
r est 0, 1 ou 2 ;
ou un stéréoisomère ou sel pharmaceutiquement acceptable de celui-ci.

6. Composé selon la revendication 5 de formule : 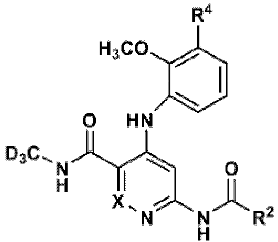 où :
X est N ou CH ;
R² est un C₁₋₆ alcoxy ;
R⁴ est absent ou un hétérocycle à 5-7 chaînons substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 2 ;
r est 0, 1 ou 2 ;
ou un stéréoisomère ou sel pharmaceutiquement acceptable de celui-ci.

7. Composé selon la revendication 6 de formule : 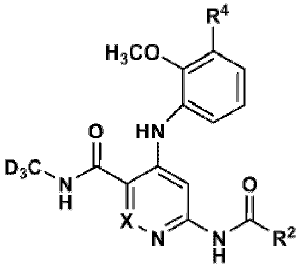 où :
X est N ou CH ;
R² est un C₁₋₆ alcoxy ;
R⁴ est absent ou un groupe triazole, thiazole, tétrazole ou oxadiazole, chacun étant substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 2 ;
r est 0, 1 ou 2 ;
ou un stéréoisomère ou sel pharmaceutiquement acceptable de celui-ci.

8. Composé selon la revendication 1 de formule : 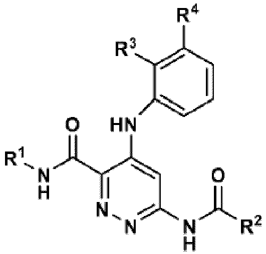 où :
R¹ est H, CD₃, un C₁₋₃ alkyle ou un C₃₋₆ cycloalkyle ;
R² est un C₁₋₆ alcoxy ;
R³ est un C₁₋₃ alkyle, un C₁₋₃ alcoxy, -NHS(O)ₚR^{a} ou -S(O)ₚR^{a} ;
R⁴ est absent ou un hétérocycle à 5-7 chaînons substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 1 ou 2 ;
r est 0, 1, 2, 3, 4 ou 5 ;
ou un stéréoisomère ou sel pharmaceutiquement acceptable de celui-ci.

9. Composé selon la revendication 1 de formule : 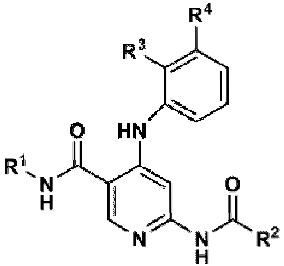 où :
R¹ est H, CD₃, un C₁₋₃ alkyle ou un C₃₋₆ cycloalkyle ;
R² est un C₁₋₆ alcoxy ;
R³ est un C₁₋₃ alkyle, un C₁₋₃ alcoxy, -NHS(O)ₚR^{a} ou -S(O)ₚR^{a} ;
R⁴ est absent ou un hétérocycle à 5-7 chaînons substitué par 0-3 R⁵ ;
R⁵ est H, un halogène, un C₁₋₃ alkyle, un C₁₋₃ alkyl-C₁₋₃ alcoxy, un -(CH₂)ᵣ-hétérocycle à 5-8 chaînons substitué par 0-2 R^{5a} ou un -CO-hétérocycle à 3-8 chaînons ;
R^{5a} est un SO₂-C₁₋₆ alkyle ou un COO-C₁₋₆ alkyle ;
R^{a} est un C₁₋₃ alkyle ou un C₃₋₆ cycloalkyle ;
p est 1 ou 2 ;
r est 0, 1, 2, 3, 4 ou 5 ;
ou un stéréoisomère ou sel pharmaceutiquement acceptable de celui-ci.

10. Composé selon la revendication 1 qui est un des composés suivants : 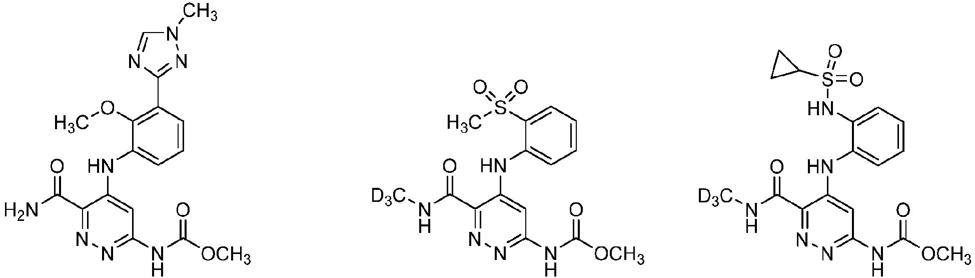 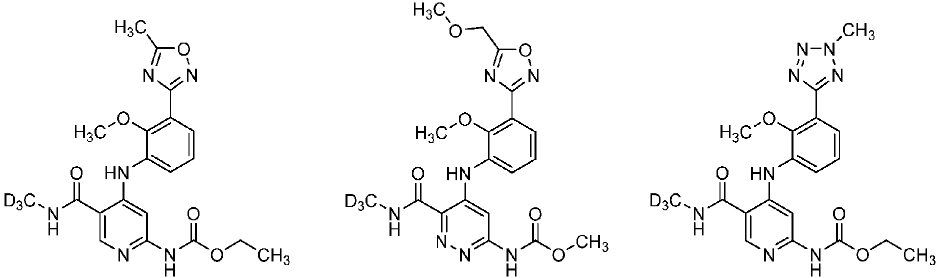 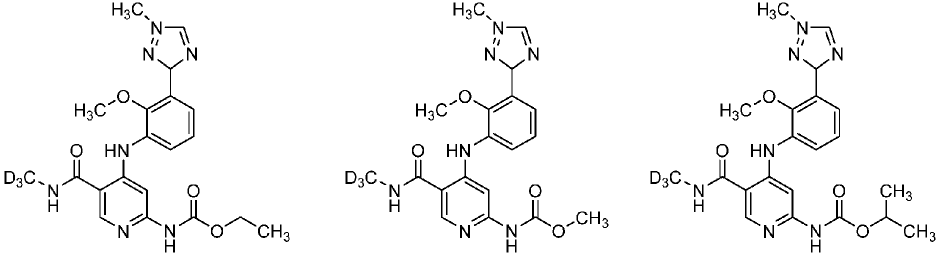 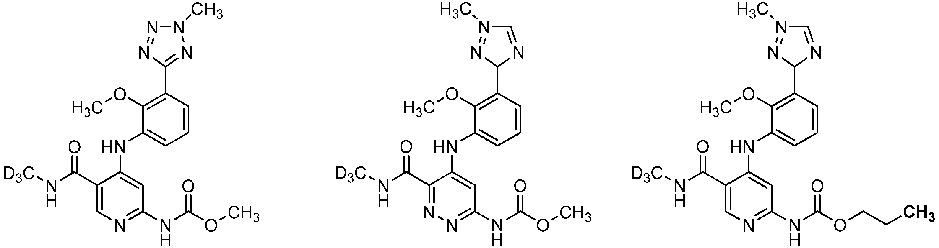 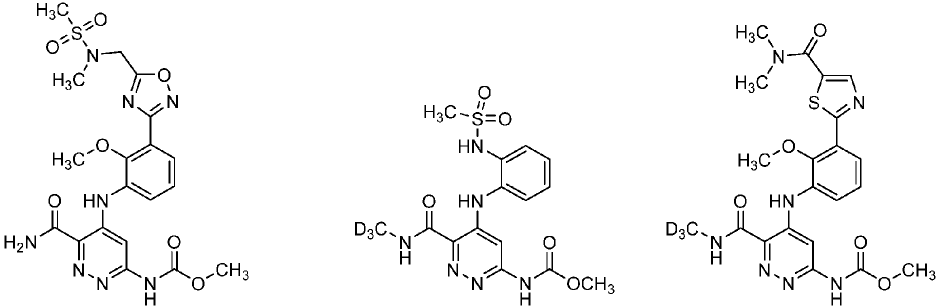 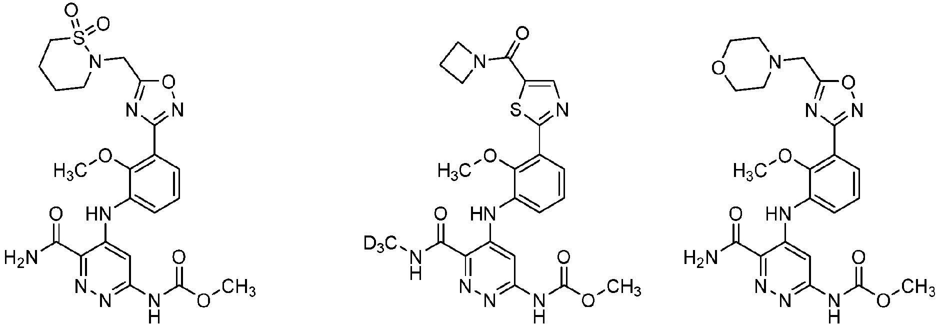 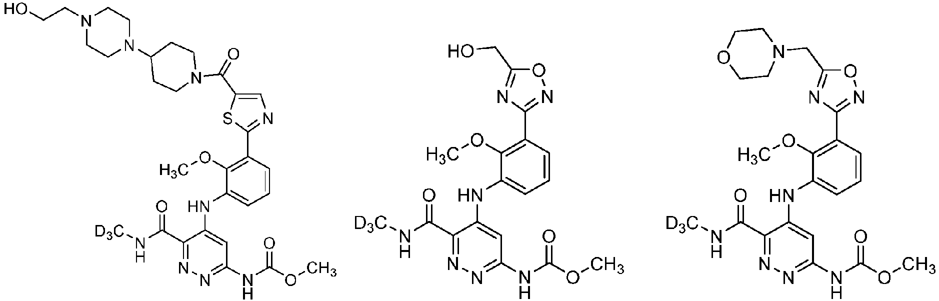 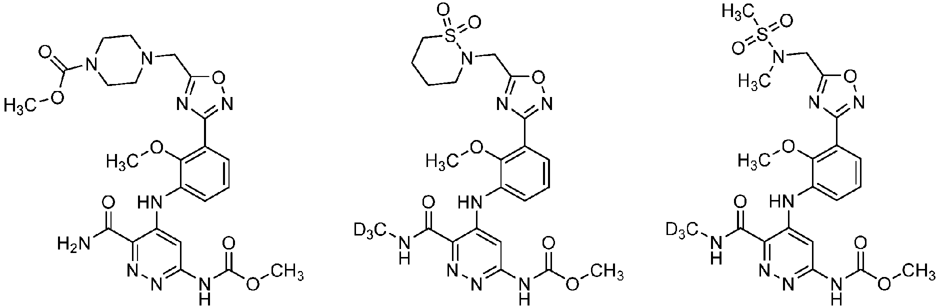 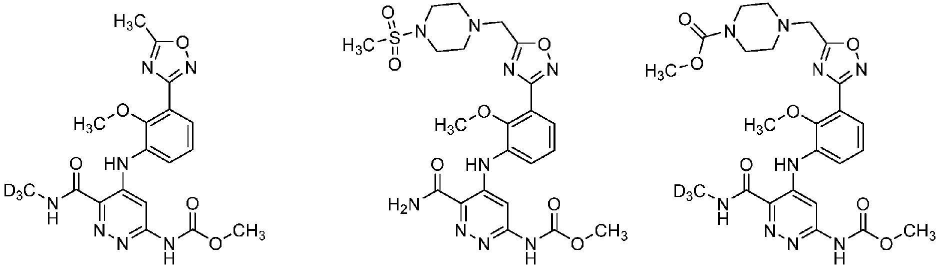 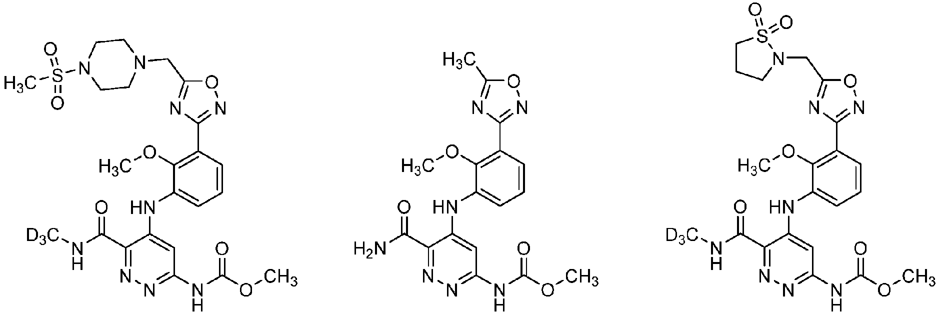 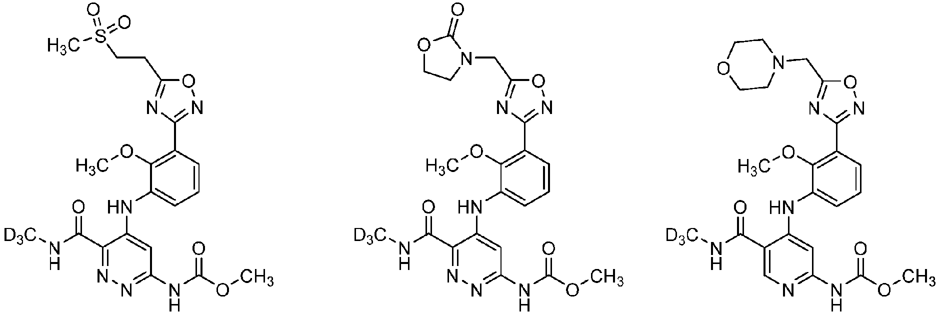 et

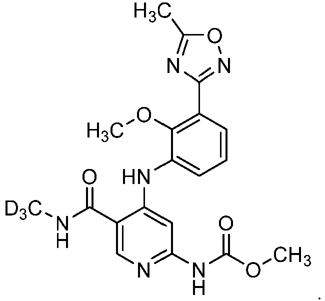

11. Composition pharmaceutique comprenant un ou plusieurs composés selon l'une quelconque des revendications 1 à 10 et un véhicule ou diluant pharmaceutiquement acceptable.

12. Composé selon l'une quelconque des revendications 1 à 10 pour utilisation en thérapie.

13. Composé selon l'une quelconque des revendications 1 à 10 pour utilisation dans une méthode de traitement d'une maladie, où la maladie est une maladie inflammatoire ou auto-immune.

14. Composé pour utilisation selon la revendication 13, où la maladie inflammatoire ou auto-immune est une sclérose en plaques, une polyarthrite rhumatoïde, une spondylite ankylosante, une maladie intestinale inflammatoire, un lupus érythémateux systémique, un psoriasis, une polyarthrite psoriasique, une maladie de Crohn, un syndrome de Sjögren ou une sclérodermie.
